(19) **Europäisches Patentamt / European Patent Office / Office européen des brevets**

(11) **EP 2 599 440 B1**

(12) **EUROPEAN PATENT SPECIFICATION**

(45) Date of publication and mention
of the grant of the patent:
**22.08.2018 Bulletin 2018/34**

(21) Application number: **11840154.6**

(22) Date of filing: **11.11.2011**

(51) Int Cl.:
*A61B 8/14* (2006.01)　　*A61B 8/00* (2006.01)
*G01S 7/52* (2006.01)　　*G01S 7/527* (2006.01)
*G01S 15/89* (2006.01)

(86) International application number:
**PCT/JP2011/076026**

(87) International publication number:
**WO 2012/063928 (18.05.2012 Gazette 2012/20)**

(54) **ULTRASONIC OBSERVATION DEVICE, METHOD FOR OPERATING ULTRASONIC OBSERVATION DEVICE, AND OPERATION PROGRAM FOR ULTRASONIC OBSERVATION DEVICE**

ULTRASCHALLBEOBACHTUNGSVORRICHTUNG, BETRIEBSVERFAHREN FÜR DIE ULTRASCHALLBEOBACHTUNGSVORRICHTUNG UND BETRIEBSPROGRAMM FÜR DIE ULTRASCHALLBEOBACHTUNGSVORRICHTUNG

DISPOSITIF D'OBSERVATION ÉCHOGRAPHIQUE, PROCÉDÉ DE FONCTIONNEMENT D'UN DISPOSITIF D'OBSERVATION ÉCHOGRAPHIQUE, ET PROGRAMME DE FONCTIONNEMENT POUR DISPOSITIF D'OBSERVATION ÉCHOGRAPHIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.11.2010 JP 2010253286**

(43) Date of publication of application:
**05.06.2013 Bulletin 2013/23**

(73) Proprietor: **Olympus Corporation**
**Tokyo 192-8507 (JP)**

(72) Inventor: **EDA, Hirotaka**
**Hachioji-shi,**
**Tokyo 192-8507 (JP)**

(74) Representative: **Gunzelmann, Rainer**
**Wuesthoff & Wuesthoff**
**Patentanwälte PartG mbB**
**Schweigerstraße 2**
**81541 München (DE)**

(56) References cited:
WO-A1-2005/122906　　CA-A1- 2 511 629
JP-A- 62 167 542　　JP-A- H05 317 312
JP-A- H05 333 003　　JP-A- 2004 310 639
JP-A- 2005 110 833　　JP-A- 2006 524 115

US-A- 4 509 524　　US-A- 5 911 160
US-A1- 2006 052 704　　US-A1- 2006 064 014
US-A1- 2006 064 014　　US-A1- 2007 006 651
US-A1- 2010 249 590

• MEGHNA SAREEN ET AL: "Normalization and backscatter spectral analysis of human carotid arterial data acquired using a clinical linear array ultrasound imaging system", ENGINEERING IN MEDICINE AND BIOLOGY SOCIETY, 2008. EMBS 2008. 30TH ANNUAL INTERNATIONAL CONFERENCE OF THE IEEE, IEEE, PISCATAWAY, NJ, USA, 20 August 2008 (2008-08-20), pages 2968-2971, XP031508626, ISBN: 978-1-4244-1814-5
• KATOUZIAN A ET AL: "Challenges in Atherosclerotic Plaque Characterization With Intravascular Ultrasound (IVUS): From Data Collection to Classification", IEEE TRANSACTIONS ON INFORMATION TECHNOLOGY IN BIOMEDICINE, IEEE SERVICE CENTER, LOS ALAMITOS, CA, US, vol. 12, no. 3, 1 May 2008 (2008-05-01), pages 315-327, XP011345471, ISSN: 1089-7771, DOI: 10.1109/TITB.2007.912352

- **YASUYOSHI SAITO ET AL.: 'Measurement of Ultrasonic Backscattering Property Caused by Change in the Scatterer's Radius for Assessment of Red Blood Cell Aggregation' TECHNICAL REPORT OF IEICE September 2008, pages 25 - 28, XP008170522**
- **M.SAREEN ET AL.: 'Normalization and backscatter spectral analysis of human carotid arterial data acquired using a clinical linear array ultrasound imaging system.' CONF.PROC.IEEE ENG.MED.BIOL.SOC. vol. 2008, 2008, pages 2968 - 2971, XP031347360**
- **A.KATOUZIAN ET AL.: 'Challenges in atherosclerotic plaque characterization with intravascular ultrasound (IVUS): from data collection to classification.' IEEE TRANS. INF.TECHNOL.BIOMED. vol. 12, no. 3, May 2008, pages 315 - 327, XP011202314**

**Description**

Field

The present invention relates to an ultrasonic observation apparatus that allows an observation of tissues of a subject by using ultrasonic waves, an operation method of the ultrasonic observation apparatus, and an operation program of the ultrasonic observation apparatus.

Background

Conventionally, a technique known as an ultrasound elastography has been known as a technique using ultrasonic waves for an examination of a breast cancer and the like (see Patent Literature 1, for example). The ultrasound elastography is a technique of utilizing a diagnostic that tissues developing a cancer or a tumor in an organism vary in hardness depending on a development status of a disease or on an individual. In this technique, an amount of strain and a modulus of elasticity of biological tissues in an examination site are measured by using ultrasonic waves under a condition where a compression is applied externally on the examination site and a result of the measurement is displayed as a cross-sectional image.

Patent Literature 2 and Patent Literature 3 disclose a method and a system for determining a material property of an object in which a measured ultrasonic interaction signal of the object in response to a broadband ultrasonic pulse is compared with a reference signal. The measured ultrasonic interaction signal is transformed from the time domain to the frequency domain to obtain an amplitude spectrum, which is compared with a reference amplitude spectrum to obtain an attenuation spectrum. The reference amplitude spectrum has low attenuation and exhibits equivalent diffraction properties as the object with respect to the broadband ultrasonic pulse. The attenuation spectrum is used to determine an attenuation parameter, which again is used to determine a material property of the object that depends on the attenuation.

Patent Literature 4 discloses an ultrasonic diagnosis apparatus and an ultrasonic image generating method for generating an attenuation image representing attenuation of an ultrasonic wave propagating in an object. A composite ultrasonic wave which comprises a first ultrasonic wave having a first center frequency and a second ultrasonic wave having a second center frequency is transmitted at least twice in the object while modulating a phase, and an echo signal corresponding to each of the at least two transmissions is received. From the echo signal, harmonics are cancelled out by performing subtraction processing between the two echo signals respectively corresponding to the at least two transmissions, and a first echo signal corresponding to the first ultrasonic wave and a second echo signal corresponding to the second ultrasonic wave are extracted. The first echo signal and the second echo signal are used for generating the attenuation image.

Patent Literature 5 discloses a radiotherapy treatment monitoring method which comprises obtaining a baseline ultrasound scan of a treatment area of a patient and subsequently obtaining one or more treatment ultrasound scans of the treatment area at various times during a course of treatment sessions. By comparing at least one of the one or more treatment ultrasound scans to the baseline ultrasound scan, a damage map is constructed representing cell damage within the treatment area.

Citation List

Patent Literature

Patent Literature 1: International Publication No. 2005/122906
Patent Literature 2: US Publication No. 2007/0006651
Patent Literature 3: Canadian Publication No. 2511629
Patent Literature 4: US Publication No. 2010/0249590
Patent Literature 5: US Publication No. 2006/0064014

Summary

Technical Problem

However, there is a problem with the above-mentioned ultrasonic elastography in that it is difficult to transmit the pressure to lower parts of vessel channels, such as blood vessels and lymph vessels. Therefore, if a tumor is formed near a vessel channel, because the boundary of the tumor is unclear, it is difficult to determine invasion of the tumor into the vessel channel. Thus, with the ultrasonic elastography, accurate determination of tissue properties is impossible in some cases.

Moreover, there is another problem with the ultrasonic elastography in that, because the pressure and the compression rate at which an examiner presses the examination site vary depending on the examiner, the reliability of measured results is low.

The present invention has been achieved to solve the problems and it is an object of the present invention to provide an ultrasonic observation apparatus, an operation method of the ultrasonic observation apparatus, and an operation program of the ultrasonic observation apparatus that enable accurate determination of the tissue properties and enhance the reliability for the determination result.

Solution to Problem

The invention is defined in claims 1, 13, 14. Further aspects and preferred embodiments are defined in the appended claims 2-12. Aspects, embodiments and examples of the present disclosure which do not fall under the scope of the appended claims do not form part of the invention and are merely provided for illustrative purposes.

To solve the above problems and achieve the above object, an ultrasonic observation apparatus according to the present invention transmits an ultrasonic wave to a subject and receives the ultrasonic wave reflected by the subject. The ultrasonic observation apparatus includes a storage unit that stores a reference spectrum that is obtained based on a frequency of an ultrasonic wave received from a reference reflector; a frequency analyzer that calculates a frequency spectrum by analyzing a frequency of the received ultrasonic wave; a frequency band setting unit that sets a frequency band that is used to approximate the frequency spectrum calculated by the frequency analyzer; a corrected frequency spectrum calculator that calculates a corrected frequency spectrum by correcting the frequency spectrum calculated by the frequency analyzer based on the reference spectrum that is stored in the storage unit; and a feature data extracting unit that extracts feature data of the subject by performing, on the corrected frequency spectrum calculated by the corrected frequency spectrum calculator, an approximating process and an attenuation correcting process in which a contribution of an attenuation which arises in a transmission of an ultrasonic wave depending on a reception depth and a frequency of the ultrasonic wave is reduced.

Moreover, in the above invention, the ultrasonic observation apparatus according to the present invention is characterized in that the storage unit stores the reference spectrum at least for each reception depth of the ultrasonic wave, and the corrected frequency spectrum calculator calculates the corrected frequency spectrum by calculating a difference between the reference spectrum and the frequency spectrum for each reception depth.

Moreover, in the above invention, the ultrasonic observation apparatus according to the present invention is characterized in that the feature data extracting unit includes an approximating unit that extracts before-correction feature data before the attenuation correcting process is performed by performing the approximating process on the frequency spectrum calculated by the frequency analyzer; and an attenuation corrector that extracts the feature data of the frequency spectrum by performing the attenuation correcting process on the before-correction feature data extracted by the approximating unit.

Moreover, in the above invention, the ultrasonic observation apparatus according to the present invention is characterized in that the feature data extracting unit includes an attenuation corrector that performs the attenuation correcting process on the frequency spectrum; and an approximating unit that extracts the feature data of the frequency spectrum by performing the attenuation correcting process on the frequency spectrum corrected by the attenuation corrector.

Moreover, in the above invention, the ultrasonic observation apparatus according to the present invention is characterized in that the attenuation corrector performs a larger correction as a reception depth of the ultrasonic wave is larger.

Moreover, in the above invention, the ultrasonic observation apparatus according to the present invention is characterized in that the approximating unit approximates the frequency spectrum by a polynomial expression via a regression analysis.

Moreover, in the above invention, the ultrasonic observation apparatus according to the present invention is characterized in that the approximating unit approximates the frequency spectrum by a primary expression and extracts plural kinds of feature data including at least two of a slope of the primary expression, an intercept of the primary expression, and an intensity which is defined by using the slope, the intercept, and a specific frequency included in a frequency band range of the frequency spectrum.

Moreover, in the above invention, the ultrasonic observation apparatus according to the present invention is characterized in that the storage unit stores, by associating with tissue properties of a plurality of known subjects, feature data of frequency spectra extracted based on ultrasonic waves reflected by the plurality of respective known subjects. The ultrasonic observation apparatus includes a tissue property determining unit that determines a tissue property in a predetermined area of the subject by using the feature data stored by being associated with the plurality of known subjects by the storage unit and the feature data extracted by the feature data extracting unit.

Moreover, in the above invention, the ultrasonic observation apparatus according to the present invention is characterized in that the storage unit stores an average of each kind of feature data in groups classified for each tissue property of the plurality of known subjects, and the tissue property determining unit sets a feature data space whose component is at least one of the plural kinds of feature data, and determines the tissue property of the subject based on a distance, on the feature data space, between a subject point having, as a coordinate in the feature data space, the feature data which is the component of the feature data space among the feature data of the frequency spectrum of the subject and a known subject average point having, as a coordinate in the feature data space, an average of the feature data which is the component of the feature data space among respective kinds of the feature data in the groups of the plurality of known subjects.

Moreover, in the above invention, the ultrasonic observation apparatus according to the present invention is characterized in that the tissue property determining unit calculates a standard deviation of feature data in populations, which are formed by adding the feature data of the subject in groups classified for each tissue property of the plurality of known subjects, and determines that a tissue property corresponding to a group having feature data whose difference between the standard deviation and a standard deviation of feature data in the groups is smallest should be the tissue property of the subject.

Moreover, in the above invention, the ultrasonic observation apparatus according to the present invention is characterized in that the storage unit includes a frequency band information storage unit that stores a frequency band whose band width becomes narrower and whose maximum frequency becomes smaller as the reception depth is larger and which is defined depending on the reception depth of the ultrasonic wave, and the frequency band setting unit sets the frequency band by referring to frequency band information stored in the frequency band information storage unit.

Moreover, in the above invention, the ultrasonic observation apparatus according to the present invention includes an input unit that receives a setting input of the frequency band. The frequency band setting unit sets the frequency band based on information received by the input unit.

Moreover, in the above invention, the ultrasonic observation apparatus according to the present invention includes a display unit that generates visual information corresponding to feature data of the subject and displays image generated based on the generated visual information and the received ultrasonic wave.

Moreover, in the above invention, the ultrasonic observation apparatus according to the present invention is characterized in that the visual information is a variable constituting a color space.

An operation method according to the present invention is an operation method of an ultrasonic observation apparatus that transmits an ultrasonic wave to a subject and receives the ultrasonic wave reflected by the subject. The operation method includes a frequency analyzing step of calculating, by a frequency analyzer, a frequency spectrum by analyzing a frequency of a received ultrasonic wave; a frequency band setting step of setting, by a frequency band setting unit, a frequency band that is used to approximate the frequency spectrum calculated at the frequency analyzing step; a corrected frequency spectrum calculating step of calculating, by a corrected frequency spectrum calculator, a corrected frequency spectrum by correcting the frequency spectrum calculated at the frequency analyzing step based on a reference spectrum that has been read from a storage unit in which a reference spectrum that is obtained based on a frequency of an ultrasonic wave received from a reference reflector is stored; and a feature data extracting step of extracting, by a feature data extracting unit, feature data of the subject by performing, on the corrected frequency spectrum calculated at the corrected frequency spectrum calculating step, an approximating process and an attenuation correcting process in which a contribution of an attenuation which arises in a transmission of an ultrasonic wave depending on a reception depth and a frequency of the ultrasonic wave is reduced.

An operation program according to the present invention is an operation program of an ultrasonic observation apparatus that transmits an ultrasonic wave to a subject and receives an ultrasonic wave reflected by the subject. The operation program causes the ultrasonic observation apparatus to execute a frequency analyzing step of calculating, by a frequency analyzer, a frequency spectrum by analyzing a frequency of a received ultrasonic wave; a frequency band setting step of setting, by a frequency band setting unit, a frequency band that is used to approximate the frequency spectrum calculated at the frequency analyzing step; a corrected frequency spectrum calculating step of calculating, by a corrected frequency spectrum calculator, a corrected frequency spectrum by correcting the frequency spectrum calculated at the frequency analyzing step based on a reference spectrum that has been read from a storage unit in which a reference spectrum that is obtained based on a frequency of an ultrasonic wave received from a reference reflector is stored; and a feature data extracting step of extracting, by a feature data extracting unit, feature data of the subject by performing, on the corrected frequency spectrum calculated at the corrected frequency spectrum calculating step, an approximating process and an attenuation correcting process in which a contribution of an attenuation which arises in a transmission of an ultrasonic wave depending on a reception depth and a frequency of the ultrasonic wave is reduced.

Advantageous Effects of Invention

According to the present invention, an ultrasonic observation apparatus calculates a frequency spectrum by analyzing a frequency of a received ultrasonic wave, sets a frequency band that is used to approximate the frequency spectrum, corrects the frequency spectrum based on a reference spectrum that has been read out from a storage unit that stores the reference spectrum obtained based on the frequency of an ultrasonic wave received from a reference reflector, and extracts feature data of a subject by performing an approximating process on the corrected frequency spectrum and then performing a correcting process to reduce a contribution of an attenuation of an ultrasonic wave that depends on a reception depth and a frequency of the ultrasonic wave. Therefore, it is possible to clearly determine the difference in tissues without using an amount of strain and a modulus of elasticity of biological tissues. Hence, it is possible to distinguish a tissue property accurately and to enhance the reliability for the observation result.

Brief Description of Drawings

[Fig. 1] FIG. 1 is a block diagram of a configuration of an ultrasonic observation apparatus according to a first embodiment of the present invention.
[Fig. 2] FIG. 2 schematically shows frequency band information stored by the ultrasonic observation apparatus according to the first embodiment of the present invention.
[Fig. 3] FIG. 3 schematically shows an outline of a creation of a reference spectrum stored by the ultrasonic observation apparatus according to the first embodiment of the present invention.
[Fig. 4] FIG. 4 is a flowchart of an outline of a process of the ultrasonic observation apparatus according to the first embodiment of the present invention.
[Fig. 5] FIG. 5 shows an example of displaying a B-mode image in a display unit of the ultrasonic observation apparatus according to the first embodiment of the present invention.
[Fig. 6] FIG. 6 is a flowchart of an outline of a process performed by a frequency analyzer of the ultrasonic observation apparatus according to the first embodiment of the present invention.
[Fig. 7] FIG. 7 schematically shows a data array of one sound ray.
[Fig. 8] FIG. 8 shows an example (first example) of a frequency spectrum calculated by the frequency analyzer of the ultrasonic observation apparatus according to the first embodiment of the present invention.
[Fig. 9] FIG. 9 shows an example (second example) of a frequency spectrum calculated by the frequency analyzer of the ultrasonic observation apparatus according to the first embodiment of the present invention.
[Fig. 10] FIG. 10 shows an example (third example) of a frequency spectrum calculated by the frequency analyzer of the ultrasonic observation apparatus according to the first embodiment of the present invention.
[Fig. 11] FIG. 11 shows an example (fourth example) of a frequency spectrum calculated by the frequency analyzer of the ultrasonic observation apparatus according to the first embodiment of the present invention.
[Fig. 12] FIG. 12 schematically shows an outline of a corrected frequency spectrum calculating process and a feature data extracting process performed on the frequency spectrum shown in FIG. 8.
[Fig. 13] FIG. 13 schematically shows an outline of a corrected frequency spectrum calculating process and a feature data extracting process performed on the frequency spectrum shown in FIG. 9.
[Fig. 14] FIG. 14 schematically shows an outline of a corrected frequency spectrum calculating process and a feature data extracting process performed on the frequency spectrum shown in FIG. 10.
[Fig. 15] FIG. 15 schematically shows an outline of a corrected frequency spectrum calculating process and a feature data extracting process performed on the frequency spectrum shown in FIG. 11.
[Fig. 16] FIG. 16 shows a new straight line defined based on feature data obtained after performing an attenuation correction on feature data related to the straight line shown in FIG. 12.
[Fig. 17] FIG. 17 is a flowchart of an outline of a process performed by a tissue property determining unit of the ultrasonic observation apparatus according to the first embodiment of the present invention.
[Fig. 18] FIG. 18 shows an example of a feature data space set by the tissue property determining unit of the ultrasonic observation apparatus according to the first embodiment of the present invention.
[Fig. 19] FIG. 19 shows an example of displaying a determination result displaying image displayed in the display unit of the ultrasonic observation apparatus according to the first embodiment of the present invention.
[Fig. 20] FIG. 20 is an explanatory view of a result of an attenuation correcting process performed by the ultrasonic observation apparatus according to the first embodiment of the present invention.
[Fig. 21] FIG. 21 schematically shows the outline of the attenuation correcting process performed by the ultrasonic observation apparatus according to the second embodiment of the present invention.
[Fig. 22] FIG. 22 schematically shows the outline of the attenuation correcting process performed by the ultrasonic observation apparatus according to the second embodiment of the present invention.

Description of Embodiments

Exemplary embodiments of the present invention (hereinafter referred to as "embodiments") will be explained below with reference to the accompanying drawings.

(First embodiment)

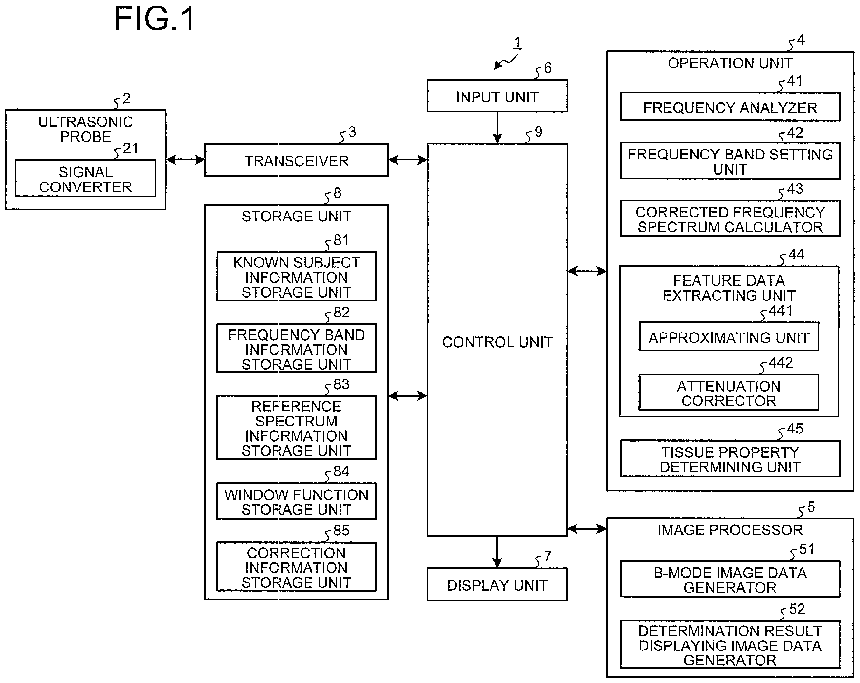

FIG. 1 is a block diagram of a configuration of an ultrasonic observation apparatus according to a first embodiment of the present invention. An ultrasonic observation apparatus 1 shown in FIG. 1 allows an observation of a subject by using ultrasonic waves.
The ultrasonic observation apparatus 1 is provided with an ultrasonic probe 2 that outputs an ultrasonic pulse to an outside and also receives an ultrasonic echo reflected at the outside, a transceiver 3 that transmits and receives an electrical signal to and from the ultrasonic probe 2, an operation unit 4 that performs a predetermined operation with respect to an electrical echo signal obtained by converting the ultrasonic echo, an image processor 5 that generates image data corresponding to the electrical echo signal obtained by converting the ultrasonic echo, an input unit 6 that is realized by using an interface such as a keyset, a mouse, and a touchscreen and accepts an input of information of various kinds, a display unit 7 that is realized by using a display panel formed by a crystal liquid or an organic EL and displays information of various kinds including images generated by the image processor 5, a storage unit 8 that stores information of various kinds including information concerning a tissue property of a known subject, and a control unit 9 that performs an operation control of the ultrasonic observation apparatus 1.
The ultrasonic probe 2 is provided with a signal converter 21 that converts the electrical pulse signal received from the transceiver 3 into an ultrasonic pulse (acoustic pulse signal) and converts the ultrasonic echo reflected by the subject outside into an electrical echo signal. The ultrasonic probe 2 may be configured such that an ultrasonic transducer mechanically scans or a plurality of ultrasonic transducers electronically scan.
The transceiver 3 is electrically connected to the ultrasonic probe 2, transmits a pulse signal to the ultrasonic probe 2, and receives an echo signal from the ultrasonic probe 2. Specifically, the transceiver 3 generates a pulse signal based on a preset waveform and transmission time and transmits the generated pulse signal to the ultrasonic probe 2. Besides, the transceiver 3 performs an A/D conversion after performing processes including amplification, filtering, and the like on the received echo signal to generate and output a digital RF signal. In the case where the ultrasonic probe 2 is configured to make a plurality of ultrasonic transducers electronically scan, the transceiver 3 includes a multichannel circuit for a beam synthesis to deal with the plurality of ultrasonic transducers.
The operation unit 4 is provided with a frequency analyzer 41 that calculates a frequency spectrum (power spectrum) of an echo signal by performing a fast Fourier transform (FFT) on the digital RF signal output from the transceiver 3, a frequency band setting unit 42 that sets a frequency band used in approximating the frequency spectrum calculated by the frequency analyzer 41, a corrected frequency spectrum calculator 43 that calculates a corrected frequency spectrum by correcting the frequency spectrum calculated by the frequency analyzer 41 based on a predetermined reference spectrum stored in the storage unit 8, a feature data extracting unit 44 that extracts feature data of a subject by performing an approximating process and an attenuation correcting process of reducing a contribution of an attenuation generated depending on a reception depth and a frequency of ultrasonic waves in the transmission of ultrasonic waves, and a tissue property determining unit 45 that determines a tissue property in a predetermined area of the subject by using the feature data extracted by the feature data extracting unit 44.
The frequency analyzer 41 calculates a frequency spectrum by performing, with respect to each sound ray (line data), the fast Fourier conversion on an FFT data group including a predetermined volume of data. A frequency spectrum shows a tendency specific to a tissue property of a subject. This is because a frequency spectrum has a correlation with size, density, acoustic impedance, and the like of a subject which is a scattering substance that scatters ultrasonic waves.
The frequency band setting unit 42 performs a frequency band setting by reading out from the storage unit 8 and referring to a frequency band table, which will be explained later, stored by the storage unit 8. The reason why the frequency band setting is changed for each reception depth in this manner is that there is a possibility in ultrasonic waves that efficient information of high frequency component is lost and inefficient information remains in an echo signal received from a site whose reception depth is large since a higher frequency component attenuates more quickly. By taking this aspect into consideration, a frequency band is set in the first embodiment so that a band width becomes narrower and a maximum frequency becomes smaller as a reception depth is larger.
The corrected frequency spectrum calculator 43 reads out from the storage unit 8 and refers to reference spectrum information, which will be explained later, stored in the storage unit 8, calculates a difference between the reference spectrum and a frequency spectrum for each reception depth, and calculate a corrected frequency spectrum.

The reason why the correction of the frequency spectrum is performed for each reception depth is the same as the reason for the setting of the frequency band explained above.

The feature data extracting unit 44 is provided with an approximating unit 441 that calculates, by performing an approximating process on the corrected frequency spectrum calculated by the corrected frequency spectrum calculator 43, before-correction feature data before an attenuation correcting process is performed, and an attenuation corrector 442 that performs the attenuation correcting process on the before-correction feature data approximated by the approximating unit 441 to extract feature data.

The approximating unit 441 approximates a frequency spectrum by a primary expression via a regression analysis to extract the before-correction feature data which defines the approximate primary expression. Specifically, the feature data extracting unit 44 calculates a slope $a_0$ and an intercept $b_0$ of the primary expression via the regression analysis and also calculates intensity at a specific frequency within a frequency band in the frequency spectrum as the before-correction feature data. While the approximating unit 441 is configured to calculate an intensity (Mid-band fit) "$c_0 = a_0 f_{MID} + b_0$" in a middle frequency "$f_{MID} = (F_{LOW} + f_{HIGH})/2$" in the first embodiment, this is just one example. The "intensity" here indicates any one of parameters such as a voltage, an electric power, a sound pressure, and an acoustic energy.

Among the feature data of three kinds, the slope $a_0$ has a correlation with a size of a scattering substance that scatters ultrasonic waves and it is considered that the slope has a smaller value as the scattering substance is larger in size in general. Besides, the intercept $b_0$ has a correlation with a size of the scattering substance, a difference in acoustic impedance, density (consistency) of the scattering substance, and the like. Specifically, the intercept $b_0$ is considered to have a larger value as the scattering substance is larger in size, to have a larger value as a value for the acoustic impedance is larger, and to have a larger value as a value for the density (consistency) of the scattering substance is larger. The intensity $c_0$ in the middle frequency $f_{MID}$ (hereinafter simply referred to as "intensity") is an indirect parameter obtained from the slope $a_0$ and the intercept $b_0$ and provides spectrum intensity in the middle within an efficient frequency band. Therefore, the intensity $c_0$ is considered to have a certain level of correlation with a brightness of the B-mode image in addition to the size of the scattering substance, the difference in acoustic impedance, and the density of the scattering substance. Here, an approximating polynomial calculated by the feature data extracting unit 44 is not limited to the primary expression and an approximating polynomial of quadratic or higher expression may be used.

A correction performed by the attenuation corrector 442 will be explained. An attenuation amount A of ultrasonic waves can be expressed as follows:

$$A = 2\alpha z f \qquad (1)$$

Here, a symbol "$\alpha$" indicates an attenuation rate, a symbol "z" indicates a reception depth of ultrasonic waves, and a symbol "f" indicates a frequency. As evidenced by expression (1), the attenuation amount A is proportional to the frequency f. A specific value for the attenuation rate $\alpha$ is 0 to 1.0(dB/cm/MHz), more preferably 0.3 to 0.7(dB/cm/MHz) in a case of a biological body, and the value is determined depending on the kind of an organ as an observation target. For example, in a case where an organ as an observation target is a pancreas, "$\alpha = 0.6$(dB/cm/MHz)" is determined. Here in the first embodiment, it is also possible to make a configuration such that the value for the attenuation rate $\alpha$ can be changed by an input from the input unit 6.

The attenuation corrector 442 corrects the before-correction feature data (the slope $a_0$, the intercept $b_0$, and the intensity $c_0$) extracted by the approximating unit 441 as follows.

$$a = a_0 + 2\alpha z \qquad (2)$$

$$b = b_0 \qquad (3)$$

$$c = c_0 + 2\alpha z f_{MID} \ (= a f_{MID} + b) \qquad (4)$$

As evidenced by expressions (2) and (4), the attenuation corrector 422 performs a correction whose correction amount is larger as the reception depth z of ultrasonic waves is larger. Besides, according to expression (3), a correction concerning to the intercept is an identical transformation. This is because the intercept is a frequency component corresponding to the frequency 0 (Hz) and is not subject to the attenuation.

The tissue property determining unit 45 calculates an average and a standard deviation of feature data of the frequency spectrum extracted by the feature data extracting unit 44 for each feature data. The tissue property determining unit 45 determines a tissue property of a predetermined area of the subject by using the calculated average and the standard deviation and an average and a standard deviation of feature data, stored in the storage unit 8, of a frequency spectrum of a known subject. The "predetermined area" here means an area in an image specified, via the input unit 6, by an operator of the ultrasonic observation apparatus 1 who watches images generated by the image processor 5 (hereinafter referred to as "area of interest"). Besides, the "the tissue property" here means any one of a cancer, an endocrine tumor, a mucinous tumor, normal tissues, and a vascular channel, for example. In the case where the subject is a pancreas, a chronic pancreatitis, an autoimmune pancreatitis, and the like are included as the tissue property.

The average and the standard deviation of the feature data calculated by the tissue property determining unit 45 reflect changes at a cellular level such as an enlargement of a nucleus and a heteromorphy and changes in tissues such as a fibrous growth in interstitium and a fibrosis substituted with parenchymal tissues, and indicate a value specific to each tissue property. Therefore, it becomes possible to accurately determine a tissue property in a predetermined area of the subject by using the average and the standard deviation of the feature data.

The image processor 5 is provided with a B-mode image data generator 51 that generates B-mode image data for performing a display by converting an amplitude of an echo signal into a brightness and a determination result displaying image data generator 52 that generates a determination result displaying image data for performing a display of a determination result of the tissue property in the area of interest and information related to the determination result by using the data output by the B-mode image data generator 51 and the operation unit 4.

The B-mode image data generator 51 generates B-mode image data by performing a signal process using known techniques such as a band-pass filter, a logarithmic transformation, a gain process, and a contrast process on the digital signal, and also culling data depending on a data step width which is determined in accordance with a display range of an image in the display unit 7.

The determination result displaying image data generator 52 generates determination result displaying image data including the determination result of the tissue property in the area of interest and a tissue property emphasized image in which the tissue property is emphasized by using the B-mode image data generated by the B-mode image data generator 51, the feature data extracted by the feature data extracting unit 44, and the determination result determined by the tissue property determining unit 45.

The storage unit 8 is provided with a known subject information storage unit 81 that stores information of a known subject, a frequency band information storage unit 82 that stores frequency band information determined depending on a reception depth of ultrasonic waves, a reference spectrum information storage unit 83 that stores reference spectrum information depending on a reception depth of ultrasonic waves, a window function storage unit 84 that stores a window function which is used in a frequency analyzing process performed by the frequency analyzer 41, and a correction information storage unit 85 that stores correction information which is referred to when an attenuation corrector 442 performs the process.

The known subject information storage unit 81 stores, by associating with a tissue property of a known subject, feature data of a frequency spectrum extracted with respect to the known subject. Besides, the known subject information storage unit 81 stores, with respect to the feature data of the frequency spectrum related to the known subject, an average and a standard deviation calculated for each of groups classified based on tissue properties of known subjects, together with the data of all kinds of the feature data of the known subjects. Here, the feature data of the known subjects is extracted in the same process as the first embodiment. It should be noted that it is not necessary to perform the process of extracting feature data of the known subjects in the ultrasonic observation apparatus 1. It is preferable that information of the known subjects stored in the known subject information storage unit 81 has a high degree of reliability on tissue property.

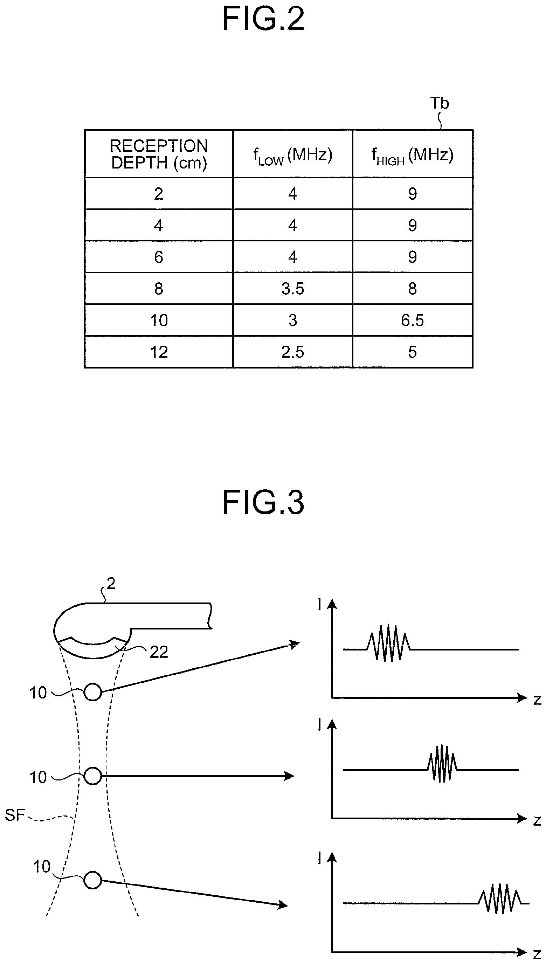

FIG. 2 schematically shows a frequency band table as frequency band information stored in the frequency band information storage unit 82. A frequency band table Tb in FIG. 2 shows a minimum frequency ($f_{LOW}$) and a maximum frequency ($f_{HIGH}$) for each reception depth of ultrasonic waves. In the frequency band table Tb, the larger the reception depth is, the narrower a band width $f_{HIGH}$-$f_{LOW}$ is and the smaller the maximum frequency $f_{HIGH}$ is. Besides, when the reception depth is relatively small (2 to 6 cm in FIG. 2), the frequency band is not changed in the frequency band table Tb since an influence of an attenuation is small. In contrast, when the reception depth is relatively large (8 to 12 cm in FIG. 2), the band is made narrow and made to shift to a side of a lower frequency since an influence of an attenuation is large. By using the frequency band table Tb, it is possible to perform imaging by extracting only a signal having efficient information. Here, the frequency band table is set individually for each kind (model) of the ultrasonic probe 2.

The reference spectrum information storage unit 83 stores, as frequency information depending on each reception depth of ultrasonic waves on a predetermined reference reflector, a frequency spectrum calculated based on an echo signal obtained by being reflected by the reference reflector (hereinafter referred to as "reference spectrum"). The reference reflector is an ideal reflector on which ultrasonic waves do not scatter, through which ultrasonic waves do not pass, and by which ultrasonic waves are not absorbed, for example. The reference spectrum is calculated for each kind of the ultrasonic probe 2 and for each reception depth of ultrasonic waves. Here, the reason why the reference spectrum is calculated for each of different ultrasonic probes 2 is that a transducer differs depending on the kind of the ultrasonic probes 2 and therefore there is a difference in a waveform of pulse to be transmitted. Here, it is not necessary that the reference reflector is the ideal reflector in a sense explained above.

FIG. 3 schematically shows an outline of a process of creating the reference spectrum. As shown in FIG. 3, a transducer 22 provided in the ultrasonic probe 2 forms a sound field (SF) nearly symmetric with respect to a travelling direction (vertical direction in FIG. 3) of ultrasonic waves around a focal point. FIG. 3 illustrates a relation between a reception depth z and an intensity I of each echo signal obtained by the ultrasonic probe 2 when a reference reflector 10 is arranged at three points including the focal point. A reference spectrum is calculated via a frequency analysis by the frequency analyzer 41 by using intensity data of an echo signal reflected by the reference reflector 10 in calculating the reference spectrum, and a result of the calculation is stored in the reference spectrum information storage unit 83.

The window function storage unit 84 stores at least one of window functions such as Hamming window, Hanning window, and Blackman window.

The correction information storage unit 85 stores information concerning conversion of expressions (2) to (4).

The storage unit 8 is realized by using a ROM that stores in advance an operation program of the ultrasonic observation apparatus according to the fist embodiment, a program for starting the operation system, and the like, and a RAM that stores operation parameters of various processes, data, and the like.

Components other than the ultrasonic probe 2 of the ultrasonic observation apparatus 1 having functional configuration explained above are realized by using a computer provided with a CPU having an operating function and a controlling function. The CPU provided in the ultrasonic observation apparatus 1 executes an operating process related to an operating method of the ultrasonic observation apparatus according to the first embodiment by reading out, from the storage unit 8, information memorized and stored in the storage unit 8 and programs of various kinds including the operation program of the ultrasonic observation apparatus explained above.

Here, it is possible to widely distribute the operation program of the ultrasonic observation apparatus according to the first embodiment by recording it in a computer-readable recording medium such as a hard disk, a flash memory, a CD-ROM, a DVD-ROM, and a flexible disk.

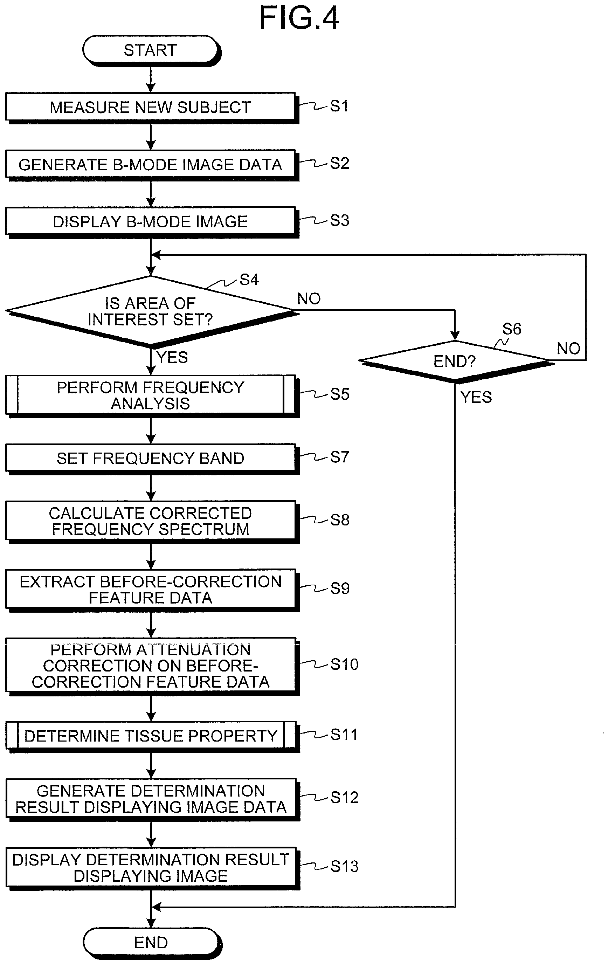

FIG. 4 is a flowchart of an outline of a process of the ultrasonic observation apparatus 1 having the configuration explained above. In FIG. 4, the ultrasonic observation apparatus 1 first performs a measurement of a new subject by the ultrasonic probe 2 (step S1). After that, the B-mode image data generator 51 generates B-mode image data (step S2).

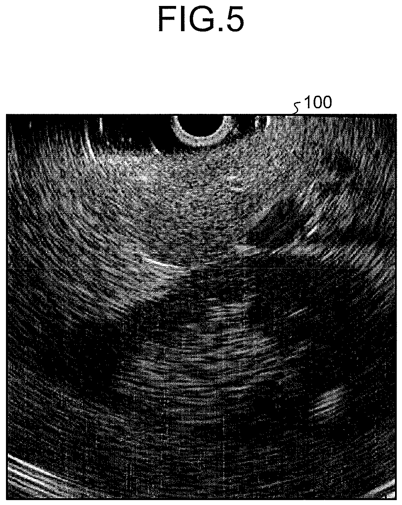

The control unit 9 then performs a control of making the display unit 7 display a B-mode image corresponding to the B-mode image data generated by the B-mode image data generator 51 (step S3). FIG. 5 shows an example of displaying a B-mode image in the display unit 7. A B-mode image 100 shown in FIG. 5 is a gray-scale image in which values for variables R (red), G (green), and B (blue), when an RGB color system is adopted for a color space, are made to conform.

After that, when an area of interest is set via the input unit 6 ("Yes" at step S4), the frequency analyzer 41 calculates a frequency spectrum by performing a frequency analysis through the FFT operation (step S5). At this step S5, it is possible to set all area of the image as the area of interest. On the other hand, when an area of interest is not set ("No" at step S4) and an instruction to end the process is input via the input unit 6 ("Yes" at step S6), the ultrasonic observation apparatus 1 ends the process. In contrast, when an area of interest is not set ("No" at step S4) and the instruction to end the process is not input ("No" at step S6), the ultrasonic observation apparatus 1 returns to step S4.

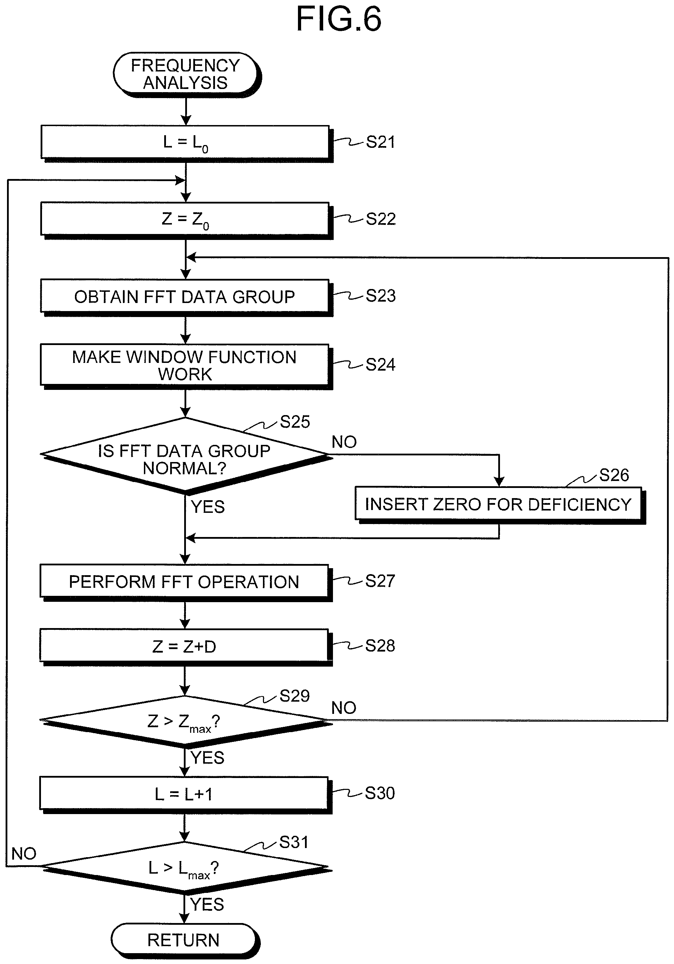

Here, the process (step S5) performed by the frequency analyzer 41 will be explained in detail with reference to the flowchart shown in FIG. 6. The frequency analyzer 41 first sets a sound ray number L of a sound ray as the first analysis target to an initial value $L_0$ (step S21). The initial value $L_0$ may be provided to a sound ray that the transceiver 3 receives for the first time, or to a sound ray corresponding to a border position at one of the left and the right of the area of interest set via the input unit 6.

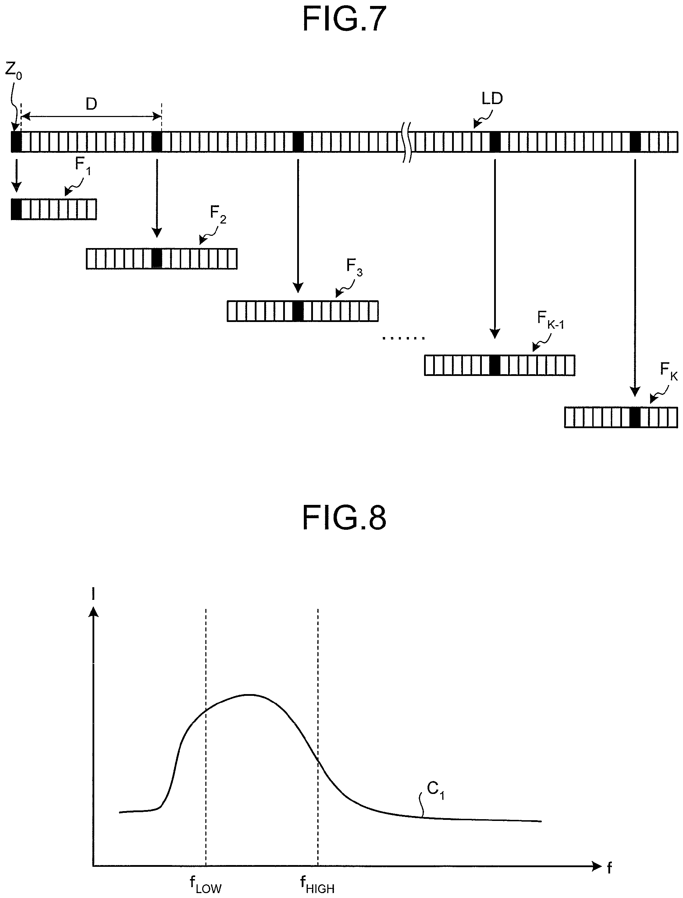

The frequency analyzer 41 then calculates all frequency spectra for a plurality of data positions set on one sound ray. The frequency analyzer 41 first sets an initial value $Z_0$ for a data position Z (corresponding to a reception depth) which represents a series of data group (FFT data group) obtained for the FFT operation (step S22). FIG. 7 schematically shows a data array of one sound ray. In the sound ray LD shown in FIG. 7, a white or a black rectangle means one piece of data. The sound ray LD is discretized by a time interval corresponding to a sampling frequency (50 MHz, for example) in the A/D conversion performed by the transceiver 3. FIG. 7 shows a case where first data piece in the sound ray LD is set to the initial value $Z_0$ for the data position Z. Here, FIG. 7 shows merely one example and a position of the initial value $Z_0$ may be arbitrarily set. For example, data position Z corresponding to an upper end position of the area of interest may be set to the initial value $Z_0$.

After that, the frequency analyzer 41 obtains FFT data group of the data position Z (step S23) and makes the window function stored in the window function storage unit 84 work on the obtained FFT data group (step S24). By making the window function work on the FFT data group, it is possible to avoid a discontinuity of the FFT data group at a border and prevent an occurrence of an artifact.

The frequency analyzer 41 then determines whether or not the FFT data group of the data position Z is a normal data group (step S25). Here, it is necessary that the FFT data group has data pieces whose number is a power of two. The number of data pieces of the FFT data group will be expressed as $2^n$ ("n" being a positive integer) below. The description "the FFT data group is normal" means that the data position Z locates at a $2^{n-1}$th position from the front in the FFT data group. In other words, the description "the FFT data group is normal" means that there is $2^{n-1} - 1$ (= N) pieces of data before the data position Z and there is $2^{n-1}$ (= M) pieces of data after the data position Z. In the case shown in FIG. 7, while FFT data groups $F_2$, $F_3$, and $F_{K-1}$ are normal, FFT data groups $F_1$ and $F_K$ are abnormal. In FIG. 7, the positive integer "n" is 4 (N = 7, M = 8).

As a result of the determination at step S25, when the FFT data group of the data position Z is normal ("Yes" at step S25), the frequency analyzer 41 moves to step S27, which will be explained later.

As a result of the determination at step S25, when the FFT data group of the data position Z is not normal ("No" at step S25), the frequency analyzer 41 generates a normal FFT data group by inserting zero for the deficiency (step S26). The FFT data group determined not to be normal at step S25 is worked on by the window function before the insertion of zero. Therefore, no discontinuity of data occurs even by inserting zero to the FFT data group. After step S26, the frequency analyzer 41 moves to step S27, which will be explained later.

At step S27, the frequency analyzer 41 obtains a frequency spectrum by performing the FFT operation by using the FFT data group (step S27).

The frequency analyzer 41 then adds a predetermined data step width D to the data position Z and calculates a data position Z of an FFT data group as a next analysis target (step S28). While it is preferable that the data step width D here is made to accord with the data step width used when the B-mode image data generator 51 generates the B-mode image data, a value larger than the data step width used by the B-mode image data generator may be set if it is requested to reduce an operation amount in the frequency analyzer 41. FIG. 7 shows a case where the data step width D is 15.

After that, the frequency analyzer 41 determines whether or not the data position Z is larger than a last data position $Z_{max}$ (step S29). Here, the last data position $Z_{max}$ may be configured to be a data length of the sound ray LD or to be a data position corresponding to a lower end of the area of interest. When the data position Z is larger than the last data position $Z_{max}$ as a result of the determination ("Yes" at step S29), the frequency analyzer 41 increases the sound ray number L by one (step S30). On the other hand, when the data position Z is not larger than the last data position $Z_{max}$ ("No" at step S29), the frequency analyzer 41 returns to step S23. In this manner, the frequency analyzer 41 performs the FFT operation on FFT data groups whose number is $[\{(Z_{max} - Z_0)/D\} + 1]$ (= K) with respect to one sound ray LD. Here, an integer [X] indicates a maximum integer not exceeding X.

When the sound ray number L after the increase at step S30 is larger than a last sound ray number $L_{max}$ ("Yes" at step S31), the frequency analyzer 41 returns to the main routine shown in FIG. 2. On the other hand, when the sound ray number L after the increase at step S30 is not more than the last sound ray number $L_{max}$ ("No" at step S31), the frequency analyzer 41 returns to step S22.

In this manner, the frequency analyzer 41 performs the FFT operation K times with respect to each of ($L_{max} - L_0 + 1$) sound rays. Here, the last sound ray number $L_{max}$ may be provided to the last sound ray that the transceiver 3 receives, or to a sound ray corresponding to a border at one of the left and the right of the area of interest, for example. A total number ($L_{max} - L_0 + 1$) $\times$ K of the FFT operations performed by the frequency analyzer 41 with respect to all the sound rays will be treated as "P" below.

After the frequency analyzing process at step S5 explained above, the frequency band setting unit 42 performs a frequency band setting for each reception depth of ultrasonic waves with reference to the frequency band table Tb stored in the frequency band information storage unit 82 (step S7). Here, the process of the frequency band setting unit 42 may be performed in parallel with the process of the frequency analyzer 41 or may be performed prior to the process of the frequency analyzer 41.

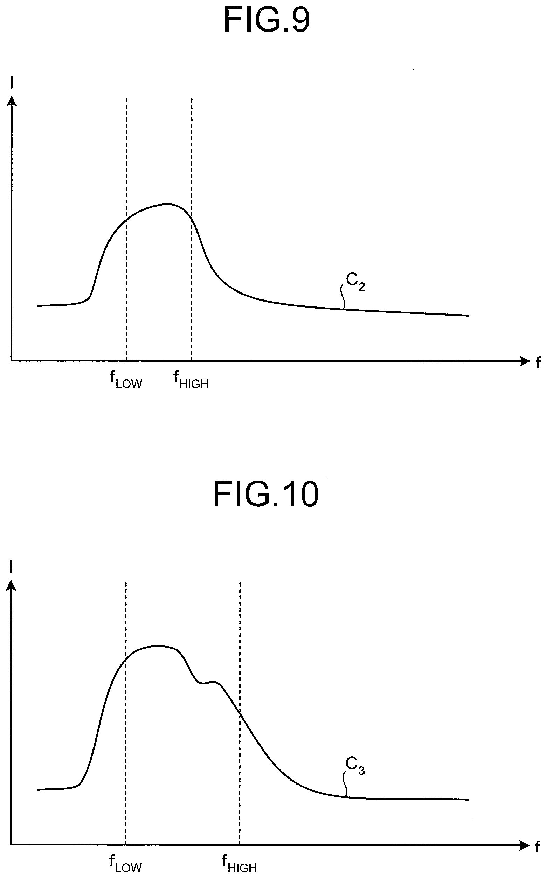

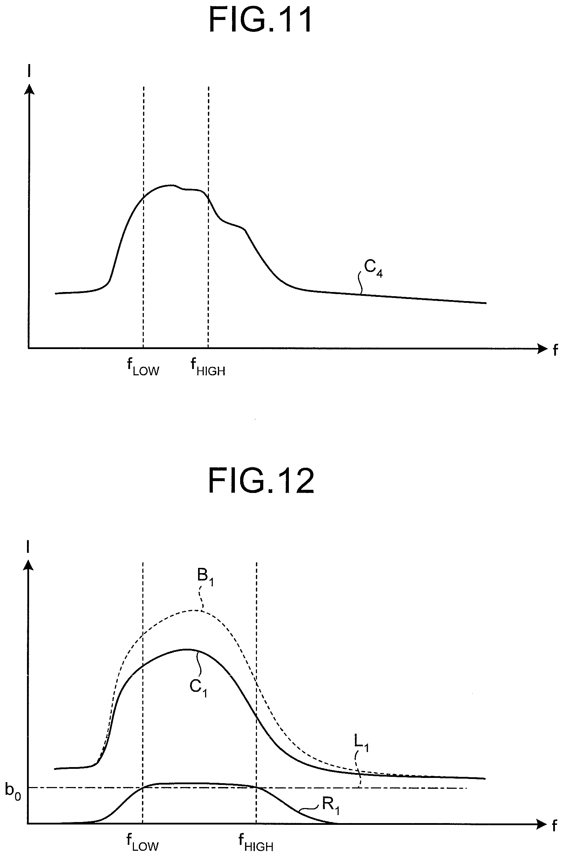

FIGS. 8 to 11 schematically show frequency spectra calculated by the frequency analyzer 41 and frequency bands set by the frequency band setting unit 42 with respect to the frequency spectra, respectively. FIGS. 8 to 11 show four kinds of frequency spectra and frequency bands with respect to a subject having the same tissue property, each one of FIGS. 8 to 11 being different from the others in at least one of the reception depth and the ultrasonic probe 2. In the figures, spectrum curves $C_1$ and $C_2$ respectively shown in FIGS. 8 and 9 show frequency spectra in respectively different reception depths when the same ultrasonic probe 2 is used. Here, the reception depth corresponding to the spectrum curve $C_1$ is smaller than the reception depth corresponding to the spectrum curve $C_2$. Besides, spectrum curves $C_3$ and $C_4$ respectively shown in FIGS. 10 and 11 show frequency spectra in respectively different reception depths when the same ultrasonic probe 2 which is, however, different from the ultrasonic probe 2 used in obtaining the spectrum curves $C_1$ and $C_2$ is used. Here, the reception depth corresponding to the spectrum curve $C_3$ is smaller than the reception depth corresponding to the spectrum curve $C_4$. Here, a curve and a straight line as a frequency function are formed by an aggregate of discrete dots in the first embodiment. In this respect, the same applies to embodiments, which will be explained later.

The reception depth corresponding to the spectrum curve $C_1$ and the reception depth corresponding to the

spectrum curve $C_3$ are the same. Besides, the reception depth corresponding to the spectrum curve $C_2$ and the reception depth corresponding to the spectrum curve $C_4$ are the same. Frequency band is defined depending on the kind of the ultrasonic probe 2. As explained above, the reception depth in the case shown in FIGS. 9 and 11 is larger than the case shown in FIGS. 8 and 10. Therefore, a band width $f_{HIGH}$-$f_{LOW}$ of a frequency band in the case shown in FIGS. 9 and 11 is narrower than the case shown in FIGS. 8 and 10.

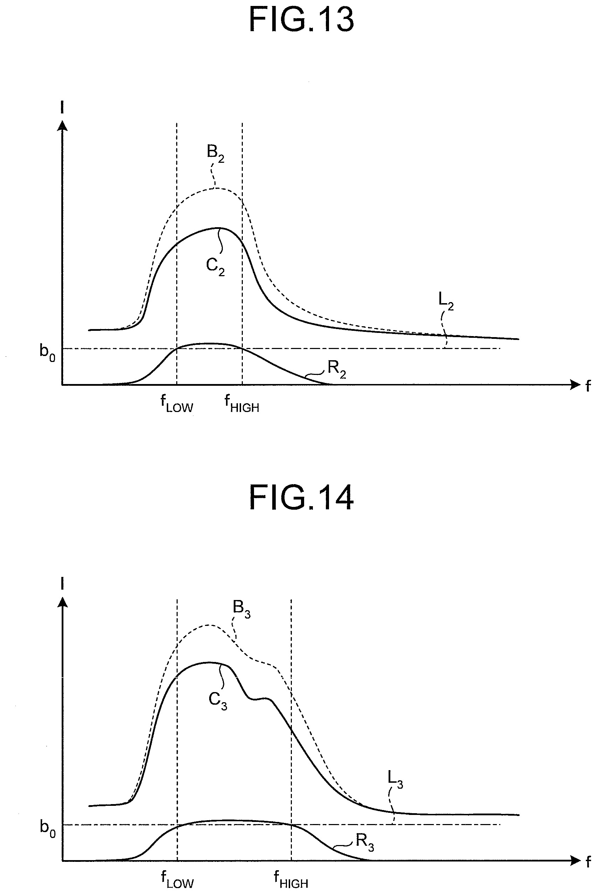

The corrected frequency spectrum calculator 43 then reads out from the reference spectrum information storage unit 83 and refers to a reference spectrum depending on the reception depth and the kind of the ultrasonic probe 2, and calculates a difference between the reference spectrum and the frequency spectrum calculated by the frequency analyzer 41 to calculate a corrected frequency spectrum (step S8.) FIGS. 12 to 15 schematically show outlines of corrected frequency spectrum calculating processes for the spectrum curves $C_1$ to $C_4$, respectively. Curves $B_1$ to $B_4$ respectively shown in FIGS. 12 to 15 show reference spectrum curves depending on the reception depth and the kind of the ultrasonic probe 2. The corrected frequency spectrum calculator 43 calculates corrected frequency spectrum curves $R_1$ to $R_4$ by taking absolute values of differences between the reference spectrum curves $B_1$ to $B_4$ and the frequency spectrum curves $C_1$ to $C_4$, respectively. Straight lines $L_1$ to $L_4$ respectively shown in FIGS. 12 to 15 will be explained in a feature data extracting process, which will be explained later.

After step S8, the approximating unit 441 extracts before-correction feature data via the regression analysis on the frequency spectra whose number is P calculated by the frequency analyzer 41 as an approximating process (step S9). Specifically, the approximating unit 441 extracts, by calculating a primary expression that approximates a frequency spectrum of a frequency band $f_{LOW} < f < f_{HIGH}$ via the regression analysis, the slope $a_0$, the intercept $b_0$, and the intensity $c_0$ which define the primary expression as before-correction feature data. Straight lines $L_1$ to $L_4$ respectively shown in FIGS. 12 to 15 are regression lines obtained by performing the regression analysis on the frequency spectrum curves $C_1$ to $C_4$, respectively at step S9. In the first embodiment, the setting of frequency band and the calculation of corrected frequency spectrum are performed prior to the extraction of feature data. Therefore, the straight lines $L_1$ to $L_4$ are just the same straight lines. In other words, feature data having the same value is extracted irrespective of the reception depth and the kind of the ultrasonic probe 2 according to the first embodiment.

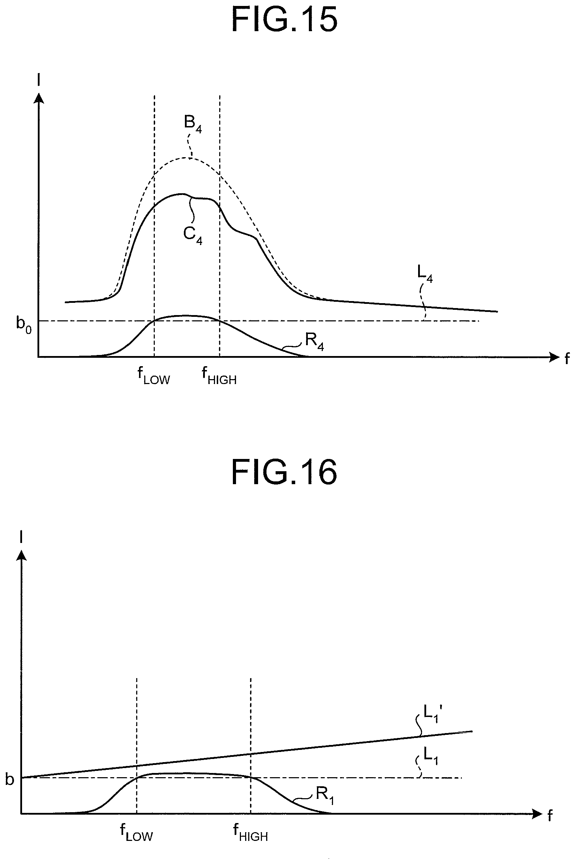

After this, the attenuation corrector 442 performs the attenuation correcting process on the before-correction feature dada extracted by the approximating unit 441 (step S10). In a case where a data sampling frequency is 50 MHz, for example, a time interval of the data sampling is 20 (nsec). Here, assuming that a sound velocity is 1530 (m/sec), an interval in distance of the data sampling is "1530 (m/sec) $\times$ 20 (nsec)/ 2 = 0.0153 (mm)". Assuming that the number of data steps from the first data piece in the sound ray LD to a data position in an FFT data group as a processing target is k, the data position Z becomes 0.0153k (mm). The attenuation corrector 442 calculates the slop a, the intercept b, and the intensity c, which are the feature data of the frequency spectrum, by substituting the value for the data position Z obtained in this manner in the reception depth z in expressions (2) to (4) explained above. FIG. 16 shows a straight line defined based on the feature data obtained after performing the attenuation correction on feature data related to the straight line $L_1$ shown in FIG. 12. An expression for a line $L_1$' shown in FIG. 16 is as follows.

$$I = af + b = (a_0 + 2\alpha Z)\ f + b_0 \qquad\qquad (5)$$

As evidenced by expression (5), the straight line $L_1$' has a slop whose inclination is large and has the same value in intercept, compared to the straight line $L_1$.

After this, the tissue property determining unit 45 determines a tissue property in the area of interest of the subject based on the feature data extracted by the feature data extracting unit 44 and the known subject information stored in the known subject information storage unit 81 (step S11).

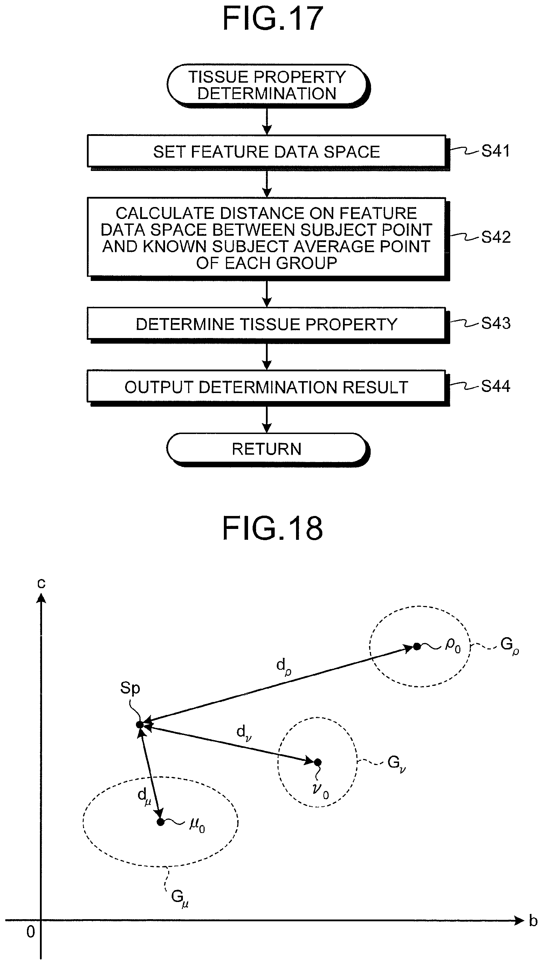

Here, the process (step S11) performed by the tissue property determining unit 45 will be explained in detail with reference to the flowchart shown in FIG. 17. The tissue property determining unit 45 first sets a feature data space used in determining a tissue property (step S41). In the first embodiment, independent parameters are two in the feature data of the three kinds, i.e., the slope a, the intercept b, and the intensity c. Therefore, it is possible to set a two-dimensional space whose components are given two kinds among the three kinds of the feature data as the feature data space. It is also possible to set one-dimensional space whose component is one kind among the three kinds of the feature data as the feature data space. While the feature data space to set is assumed to be determined in advance at step S41, a desired feature data space may be selected by the operator via the input unit 6.

FIG. 18 shows an example of the feature data space set by the tissue property determining unit 45. In the feature data space shown in FIG. 18, the horizontal axis indicates the intercept b and the vertical axis indicates the intensity c. A dot Sp shown in FIG. 18 indicates a point having, as a coordinate in the feature data space, the intercept b and the intensity c which are calculated with respect to the subject as a determination target (hereinafter referred to as "subject point"). Besides, areas $G_\mu$, $G_\nu$, and $G_\rho$ shown in FIG. 18 indicate that respective tissue properties of the known subjects stored in the known subject information storage unit 81 are $\mu$, $v$, and $p$, respectively. In the case shown in FIG. 18, the three groups $G_\mu$, $G_v$, and $G_\rho$ are present in respective areas each of which is isolated from other groups in the feature data space.

Since the classification and the determination of tissue properties also in obtaining feature data of a known subject are performed by using, as an indicator, feature data obtained via the attenuation correction on before-correction feature data of frequency spectrum obtained by the frequency analysis in the first embodiment, it is possible to clearly distinguish tissue properties which differ from each other. Especially, since feature data on which the attenuation correction is performed is used in the first embodiment, it is possible to obtain an area of each group in the feature data space in a condition where groups are separated more clearly, compared to the case of using feature data extracted without performing the attenuation correction.

After step S41, the tissue property determining unit 45 calculates distances $d_\mu$, $d_v$, and $d_\rho$, on the feature data space, between the subject point Sp and respective points $\mu_0$, $v_0$, and $\rho_0$ each of which has, as a coordinate in the feature data space, an average of intercepts b and an average of intensities c of frequency spectra in FFT data groups included in each of the groups $G_\mu$, $G_v$, and $G_\rho$, the points being hereinafter referred to as "known subject average point" (step S42). Here, when b-axis component and c-axis component in the feature data space differ significantly in scale, it is preferable to arbitrarily perform a weighting so that contributions of respective distances become nearly uniform.

The tissue property determining unit 45 then determines a tissue property of all the subject points including the subject point Sp based on the distances calculated at step S42 (step S43). For example, since the distance $d_\mu$ is the smallest in the case shown in FIG. 18, the tissue property determining unit 45 determines that the tissue property of the subject should be $\mu$. When the subject point Sp is separated away from the known subject average points $\mu_0$, $v_0$, and $\rho_0$ extremely, a degree of reliability for the determination result on the tissue property is low even if the smallest value among the distances $d_\mu$, $d_v$, and $d_\rho$ is obtained. So, when the distances $d_\mu$, $d_v$, and $d_\rho$ are larger than a predetermined threshold value, the tissue property determining unit 45 may output an error signal. Besides, when there arise two or more smallest values among the distances $d_\mu$, $d_v$, and $d_\rho$, the tissue property determining unit 45 may select all tissue properties corresponding to the smallest values each as a candidate or select any one of the tissue properties in accordance with a predetermined rule. In the latter situation, a method of placing a high priority on a tissue property whose degree of malignancy is high like a cancer can be listed. Besides, when there arise two or more smallest values among the distances $d_\mu$, $d_v$, and $d_\rho$, the tissue property determining unit 45 may output an error signal.

After this, the tissue property determining unit 45 outputs the result of the distance calculation at step S42 and the result of the determination at step S43 (step S44). Thus, the tissue property determining process at step S11 is ended.

After step S11 explained above, the determination result displaying image data generator 52 generates determination result displaying image data by using the B-mode image data generated by the B-mode image data generator 51, the feature data calculated by the feature data extracting unit 44, and the determination result determined by the tissue property determining unit 45 (step S12) .

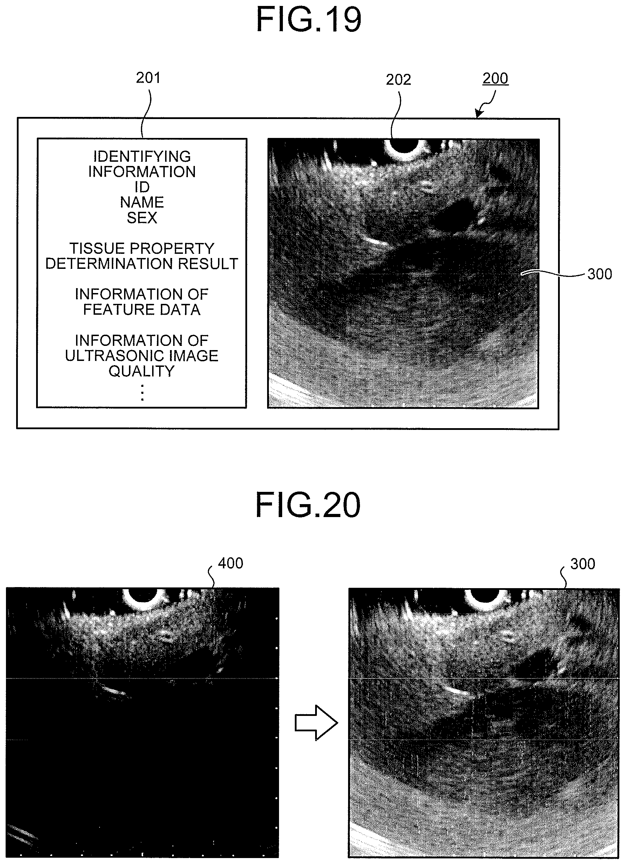

The display unit 7 then displays the determination result displaying image generated by the determination result displaying image data generator 52 (step S13). FIG. 19 shows an example of displaying a determination result displaying image displayed in the display unit 7. A determination result displaying image 200 shown in FIG. 19 includes an information displaying part 201 that displays related information of various kinds including the determination result on the tissue property and an image displaying part 202 that displays a tissue property emphasized image in which the tissue property is emphasized based on the B-mode image.

In the information displaying part 201, identifying information (ID number, name, sex, and the like) of the subject for example, the tissue property determination result obtained by the tissue property determining unit 45, information concerning the feature data in performing the tissue property determination, and information of ultrasonic image quality such as a gain and a contrast are displayed. Here, for the information concerning the feature data, it is possible to make a display utilizing an average and a standard deviation of feature data of frequency spectra of FFT data groups, the number of which is Q, present in an inside of the area of interest. Specifically, it is possible to display "Slope = 1.5 $\pm$ 0.3 (dB/MHz), Intercept = -60 $\pm$ 2 (dB), and Intensity = -50 $\pm$ 1.5 (dB)", for example in the information displaying part 201.

A tissue property emphasized image 300 displayed in the image displaying part 202 is a gray-scale image in which the intercept b is uniformly allotted to R (red), G (green), and B (blue) with respect to the B-mode image 100 shown in FIG. 5.

Due to the display of the determination result displaying image 200 having the configuration explained above by the display unit 7, it becomes possible for the operator to grasp the tissue property in the area of interest more accurately. Here, the determination result displaying image is not limited to the configuration explained above. For example, the tissue property emphasized image and the B-mode image may be displayed side by side for the determination result displaying image. Thus, it is possible to recognize the difference between the two images on one frame.

FIG. 20 is an explanatory view of a result of the attenuation correcting process performed by the ultrasonic observation apparatus 1. An image 400 shown in FIG. 20 is a tissue property emphasized image in the case where the attenuation correction is not performed. In the tissue property emphasized image 400, a signal intensity becomes lowered due to an influence of the attenuation in an area whose reception depth is large (downward area in FIG. 20) and an image becomes dark. In contrast, it is apparent that an image whose brightness is uniform over the entirety of the frame is obtained in the tissue property emphasized image 300 on which the attenuation correction is performed.

The tissue property emphasized image 300 shown in FIGS. 19 and 20 is just one example. In addition, it is possible to display the tissue property emphasized image in a color image by allotting the slope a, the intercept b, and the intensity c respectively to R (red), G (green), and B (blue), for example. In this case, since a tissue property is expressed by a specific color, it is possible for the operator to grasp the tissue property in the area of interest based on the color distribution of the image. Besides, instead of constituting a color space in the RGB color system, a color space may be constituted by variables for complementary colors such as cyan, magenta, and yellow and feature data may be allotted to respective variables. Besides, tissue property emphasized image data may be generated by mixing, by a predetermined ratio, the B-mode image data and color image data. Besides, tissue property emphasized image data may be generated by making only the area of interest replaced with color image data.

According to the first embodiment explained so far, it is possible to clearly determine the difference in tissues without using an amount of strain and a modulus of elasticity of biological tissues since a frequency spectrum is calculated by analyzing a frequency of received ultrasonic waves, a frequency band used in approximating the frequency spectrum is set, the frequency spectrum is corrected based on a reference spectrum read out from the storage unit that stores the reference spectrum obtained based on a frequency of ultrasonic waves received from the reference reflector, before-correction feature data is extracted by performing the approximating process on the corrected frequency spectrum, and then feature data of the subject is extracted by performing the attenuation correcting process in which the contribution of ultrasonic attenuation which depends on the reception depth and the frequency of ultrasonic waves is reduced. Hence, it is possible to enable distinguishing a tissue property accurately and to enhance the reliability for the observation result.

According to the first embodiment, it is possible to remove an influence of the attenuation associated with the transmission of ultrasonic waves and to perform a tissue property determination with higher accuracy since the attenuation correction is performed on the extracted feature data.

According to the first embodiment, it is possible to remove an influence of the attenuation associated with the transmission of ultrasonic waves and to perform a tissue property determination with even higher accuracy since the frequency band is determined so that the band width becomes narrower and the maximum frequency becomes smaller as the reception depth is larger.

(Second embodiment)

A second embodiment of the present invention differs from the first embodiment in the feature data extracting process performed by the feature data extracting unit. A configuration of an ultrasonic observation apparatus according to the second embodiment is the same as that of the ultrasonic observation apparatus 1 explained in the first embodiment. Therefore, an identical component corresponding to a component of the ultrasonic observation apparatus 1 will be assigned with the same reference symbol in the explanation below.

In a feature data extracting process according to the second embodiment, the attenuation corrector 442 first performs the attenuation correcting process on the corrected frequency spectrum calculated by the corrected frequency spectrum calculator 43. After that, the approximating unit 441 extracts feature data of the frequency spectrum by performing the approximating process on the corrected frequency spectrum on which the attenuation correction is performed by the attenuation corrector 442.

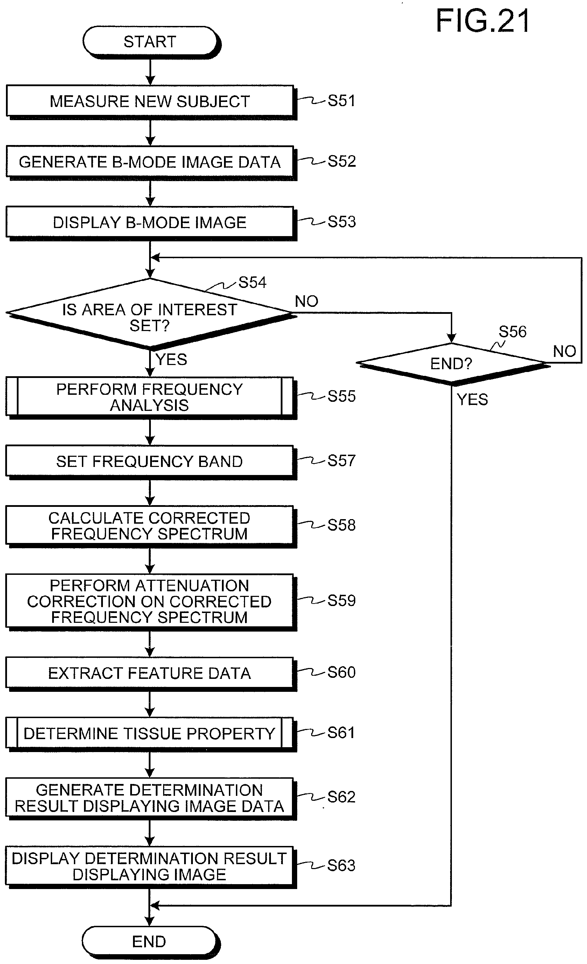

FIG. 21 is a flowchart of an outline of an attenuation correcting process performed by the ultrasonic observation apparatus according to the second embodiment. In FIG. 21, processes of steps S51 to S58 sequentially correspond to processes of steps S1 to S8 in FIG. 4.

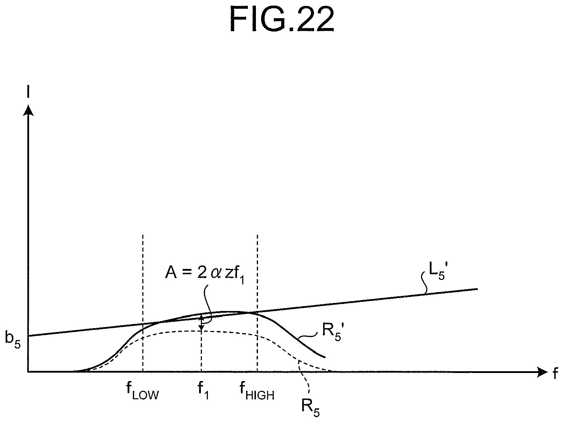

At step S59, the attenuation corrector 442 performs the attenuation correction on the corrected frequency spectrum calculated by the corrected frequency spectrum calculator 43 (step S59). FIG. 22 schematically shows an outline of the process at step S59. As shown in FIG. 22, the attenuation corrector 442 obtains a new frequency spectrum curve $R_5'$ by performing, with respect to a corrected frequency spectrum curve $R_5$, a correction in which an attenuation amount A in expression (1) explained above is added to the intensity I on all the frequencies f. Thus, it is possible to obtain a frequency spectrum in which the contribution of the attenuation associated with the transmission of ultrasonic waves is reduced.

After this, the approximating unit 441 extracts feature data of frequency spectrum via the regression analysis on all the frequency spectra on which the attenuation correction is performed by the attenuation corrector 442 (step S60). Specifically, the approximating unit 441 calculates the slope a, the intercept b, and the intensity c in the middle frequency $f_{MID}$ of the primary expression via the regression analysis. A straight line $L_5'$ shown in FIG. 22 is a regression line (intercept $b_5$) obtained by performing the feature data extracting process on the corrected frequency spectrum curve $R_5$ at step S60.

Processes of steps S61 to S63 sequentially correspond to the processes of steps S11 to S13 in FIG. 4.

According to the second embodiment of the present invention explained so far, it is possible to clearly determine the difference in tissues without using an amount of strain and a modulus of elasticity of biological tissues since a frequency spectrum is calculated by analyzing a frequency of received ultrasonic waves, a frequency band used in approximating the frequency spectrum is set, the frequency spectrum is corrected based on a reference spectrum read out from the storage unit that stores the reference spectrum obtained based on a frequency of ultrasonic waves received from the reference reflector, the attenuation correcting process in which the contribution of ultrasonic attenuation which depends on the reception depth and the frequency of ultrasonic waves is reduced is performed on the corrected frequency spectrum, and then feature data of the subject is extracted by performing the approximating process. Hence, it is possible to enable distinguishing a tissue property accurately and to enhance the reliability for the observation result.

According to the second embodiment, it is possible to remove an influence of the attenuation associated with the transmission of ultrasonic waves and to perform a tissue property determination with higher accuracy since the attenuation correction is performed on the corrected frequency spectrum.

According to the second embodiment, it is possible to remove an influence of the attenuation associated with the transmission of ultrasonic waves and to perform a tissue property determination with even higher accuracy since the frequency band is determined so that the band width becomes narrower and the maximum frequency becomes smaller as the reception depth is larger.

(Third embodiment)

A third embodiment of the present invention differs from the first embodiment in the tissue property determining process by the tissue property determining unit. A configuration of an ultrasonic observation apparatus according to the third embodiment is the same as that of the ultrasonic observation apparatus 1 explained in the first embodiment. Therefore, an identical component corresponding to a component of the ultrasonic observation apparatus 1 will be assigned with the same reference symbol in the explanation below.

The tissue property determining unit 45, after making up new populations by adding feature data (a, b, c) to each of the groups $G_\mu$, $G_v$, and $G_\rho$ (see FIG. 18) respectively for the tissue properties $\mu$, v, and p, obtains a standard deviation for each kind, constituting each tissue property, of the feature data.

After that, the tissue property determining unit 45 calculates a difference (hereinafter referred to simply as "difference in standard deviation") between a standard deviation of each kind of the feature data of the groups $G_\mu$, $G_v$, and $G_\rho$ in original populations constituted only by known subjects and a standard deviation of each kind of the feature data of the groups $G_\mu$, $G_v$, and $G_\rho$ in the new populations in which the new subject is added each, and determines that a tissue property corresponding to a group including feature data whose difference in standard deviation is the smallest should be the tissue property of the subject.

Here, the tissue property determining unit 45 may calculate the difference in standard deviation only with respect to the difference in standard deviation of given pieces of feature data selected in advance among plural pieces of feature data. The selection of the feature data in this case may be arbitrarily performed by the operator or may be automatically performed by the ultrasonic observation apparatus 1.

Besides, the tissue property determining unit 45 may calculate a value in which a weight is arbitrarily added to the difference in standard deviation of all the feature data in each group and determine that a tissue property corresponding to a group the calculated value of which is the smallest should be the tissue property of the subject. In this case, when the feature data is "slop a, intercept b, and intensity c", for example, the tissue property determining unit 45 performs, by setting weights for the slop a, the intercept b, and the intensity c, respectively to $w_a$, $w_b$ and $w_c$, a calculation of $w_a \cdot$(difference in standard deviation of a) + $w_b \cdot$(difference in standard deviation of b) + $w_c \cdot$(difference in standard deviation of c), and determines the tissue property of the subject based on the calculated value. Here, the values for the weights $w_a$, $w_b$ and $w_c$ may be arbitrarily set by the operator or may be automatically set by the ultrasonic observation apparatus 1.

Besides, the tissue property determining unit 45 may calculate the square root of a value in which a weight is arbitrarily added to the square of the difference in standard deviation of all the feature data for each group, and determine that a tissue property corresponding to a group the square root of which is the smallest should be the tissue property of the subject. In this case, when the feature data is "slop a, intercept b, and intensity c", for example, the tissue property determining unit 45 performs, by setting weights for the slop a, the intercept b, and the intensity c, respectively to $w'_a$, $w'_b$ and $w'_c$, a calculation of $\{w'_a \cdot$(difference in standard deviation of a)$^2$ + $w'_b \cdot$(difference in standard deviation of b)$^2$ + $w'_c \cdot$(difference in standard deviation of c)$^2\}^{1/2}$, and makes the tissue property determination based on the calculated value. Here in this case, too, the values for the weights $w'_a$, $w'_b$ and $w'_c$ may be arbitrarily set by the operator or may be automatically set by the ultrasonic observation apparatus 1.

According to the third embodiment of the present invention explained so far, it is possible to enable distinguishing a tissue property accurately, to enhance the reliability for the observation result, and to perform a tissue property determination with higher accuracy by removing an influence of the attenuation associated with the transmission of ultrasonic waves, similarly to the first embodiment explained above.

While the tissue property determining unit 45 determines the tissue property based on the change in standard deviation of each kind of feature data between an original population and another population in which a new subject is added in the third embodiment, this configuration is just one example. The tissue property determining unit 45 may determine the tissue property based on a change in average of each kind of the feature data between the original population and another population in which a new subject is added, for example.

While the embodiments of the present invention are explained so far, the present invention is not limited only to the first to the third embodiments explained above. In other words, the present invention may cover various embodiments within a scope not departing from the technical ideas as defined by the appended claims.

Reference Signs List

1 ultrasonic observation apparatus
2 ultrasonic probe
3 transceiver
4 operation unit
5 image processor
6 input unit
7 display unit
8 storage unit
9 control unit
21 signal converter
22 transducer
41 frequency analyzer
42 frequency band setting unit
43 corrected frequency spectrum calculator
44 feature data extracting unit
45 tissue property determining unit
51 B-mode image data generator
52 determination result displaying image data generator
81 known subject information storage unit
82 frequency band information storage unit
83 reference spectrum information storage unit
84 window function storage unit
85 correction information storage unit
100 B-mode image
200 determination result displaying image
201 information displaying part
202 image displaying part
300, 400 tissue property emphasized image
441 approximating unit
442 attenuation corrector

**Claims**

1. An ultrasonic observation apparatus (1) for transmitting an ultrasonic wave to a subject, receiving the ultrasonic wave reflected by the subject, and determining a tissue property of the subject based on an analysis result of the frequency spectrum of the received ultrasonic wave, the ultrasonic observation apparatus (1) comprising:

    a frequency analyzer (41) configured to calculate a frequency spectrum by analyzing a frequency of the received ultrasonic wave received from a specified reception depth;
    a frequency band information storage unit (82) that stores, for each reception depth, frequency values as upper limits of frequency bands in a plurality of reception depths;
    a frequency band setting unit (42) configured to set a frequency band of the frequency spectrum in the specified reception depth, calculated by the frequency analyzer (41), based on a frequency value in the specified reception depth among the frequency values stored in the frequency band information storage unit (82);

a reference spectrum information storage unit (83) that stores, for each reception depth, reference spectra in the plurality of reception depths obtained based on frequencies of ultrasonic waves received from a reference reflector;

a corrected frequency spectrum calculator (43) configured to calculate a corrected frequency spectrum by correcting the frequency spectrum calculated by the frequency analyzer (41) based on the reference spectrum in the specified reception depth among the reference spectra stored in the reference spectrum information storage unit (83); and

a feature data extracting unit (44) including an approximating unit (441) configured to perform an approximating process only on a part of the corrected frequency spectrum calculated by the corrected frequency spectrum calculator (43), the part of the corrected frequency spectrum being included in the frequency band set by the frequency band setting unit (42), the feature data extracting unit (44) being configured to extract feature data indicating the tissue property of the subject using a result of the approximating process performed by the approximating unit (441);

wherein the frequency band setting unit (42) is configured to set the frequency band which is used when the approximating unit (441) performs the approximating process.

2. The ultrasonic observation apparatus (1) according to claim 1, wherein the feature data extracting unit (44) includes an attenuation corrector (442) configured to perform an attenuation correcting process for performing an identical transformation or reducing a contribution of an attenuation which arises in a transmission of the ultrasonic wave depending on a reception depth and a frequency of the ultrasonic wave.

3. The ultrasonic observation apparatus (1) according to claim 1, wherein
the frequency band information storage unit (82) further stores, for each reception depth, frequency values as lower limits of the frequency bands in the plurality of reception depths, and
the frequency band setting unit (42) is configured to set the frequency band of the frequency spectrum calculated by the frequency analyzer, further based on a lower limit of a frequency band in the specified reception depth stored in the frequency band information storage unit (82).

4. The ultrasonic observation apparatus (1) according to claim 3, further comprising an ultrasonic probe (2) for transmitting and receiving the ultrasonic wave,
wherein the reference spectrum information storage unit (83) stores, for each reception depth according to kind of the ultrasonic probe (2), the reference spectra in the plurality of reception depths obtained based on the frequencies of the ultrasonic waves received from the reference reflector, and
the frequency band information storage unit (82) stores, for each reception depth according to kind of the ultrasonic probe (2), the upper limits and lower limits of the frequency bands.

5. The ultrasonic observation apparatus (1) according to claim 2, wherein the attenuation corrector (442) is configured to perform a larger correction as a reception depth of the ultrasonic wave is larger.

6. The ultrasonic observation apparatus (1) according to any one of claims 1 and 5, wherein the approximating unit (441) is configured to approximate the frequency spectrum by a polynomial expression via a regression analysis.

7. The ultrasonic observation apparatus (1) according to claim 6, wherein the approximating unit (441) is configured to approximate the frequency spectrum by a primary expression and extracts plural kinds of feature data including at least two of a slope of the primary expression, an intercept of the primary expression, and an intensity which is defined by using the slope, the intercept, and a specific frequency included in a frequency band range of the frequency spectrum.

8. The ultrasonic observation apparatus (1) according to claim 1, further comprising:

a known subject information storage unit (81) that stores feature data of frequency spectra extracted based on ultrasonic waves reflected by a plurality of known subjects, by associating the feature data of frequency spectra with tissue properties of the plurality of known subjects; and

a tissue property determining unit (45) configured to determine a tissue property in a specified area of the subject (2) by using the feature data stored in the known subject information storage unit (81) by being associated with the plurality of known subjects and the feature data extracted by the feature data extracting unit (44).

9. The ultrasonic observation apparatus (1) according to any one of claims 1 to 8, wherein

the frequency band information storage unit (82) stores the frequency bands in which the larger a reception depth is, the narrower a band width is and the smaller a maximum frequency value is, and the band width is defined by the maximum frequency value and a minimum frequency value.

10. The ultrasonic observation apparatus (1) according to any one of claims 1 to 9, comprising
a display unit (7) configured to generate visual information corresponding to the feature data of the subject and to display an image generated based on the generated visual information and the received ultrasonic wave.

11. The ultrasonic observation apparatus (1) according to claim 10, wherein the visual information is a variable constituting a color space.

12. The ultrasonic observation apparatus (1) according to claim 2, wherein the attenuation corrector (442) is configured to perform a correction based on an attenuation amount $A = 2\alpha zf$, where "$\alpha$" indicates an attenuation rate, "$z$" indicates a reception depth, and "$f$" indicates a frequency.

13. An operation method of an ultrasonic observation apparatus for transmitting an ultrasonic wave to a subject, receiving the ultrasonic wave reflected by the subject, and determining a tissue property of the subject based on an analysis result of a frequency spectrum of the received ultrasonic wave, the operation method comprising:

a frequency analyzing step (S5) of calculating, by a frequency analyzer, a frequency spectrum by analyzing a frequency of a received ultrasonic wave received from a specified reception depth;
a frequency band setting step (S7) of setting, by a frequency band setting unit, a frequency band of the frequency spectrum in the specified reception depth, calculated by the frequency analyzer, based on a frequency value in the specified reception depth among frequency values stored in a frequency band information storage unit that stores, for each reception depth, the frequency values as upper limits of frequency bands in a plurality of reception depths;
a corrected frequency spectrum calculating step (S8) of calculating, by a corrected frequency spectrum calculator, a corrected frequency spectrum by correcting the frequency spectrum calculated at the frequency analyzing step based on a reference spectrum in the specified reception depth that is read out from a reference spectrum information storage unit that stores, for each reception depth, reference spectra in the plurality of reception depths obtained based on frequencies of ultrasonic waves received from a reference reflector; and
a feature data extracting step (S9, S10) of performing, by a feature data extracting unit, an approximating process only on a part of the corrected frequency spectrum calculated at the corrected frequency spectrum calculating step, the part of the corrected frequency spectrum being included in the frequency band set by the frequency band setting unit, to extract feature data indicating the tissue property of the subject using a result of the approximating process,
wherein the frequency band setting step includes setting of the frequency band which is used when the approximating process is performed.

14. An operation program of an ultrasonic observation apparatus for transmitting an ultrasonic wave to a subject, receiving the ultrasonic wave reflected by the subject, and determining a tissue property of the subject based on an analysis result of a frequency spectrum of the received ultrasonic wave, the operation program causing the ultrasonic observation apparatus to execute:

a frequency analyzing step (S5) of calculating, by a frequency analyzer, a frequency spectrum by analyzing a frequency of a received ultrasonic wave received from a specified reception depth;
a frequency band setting step (S7) of setting, by a frequency band setting unit, a frequency band of the frequency spectrum in the specified reception depth, calculated at the frequency analyzing step (S5), based on a frequency value in the specified reception depth among frequency values stored in a frequency band information storage unit that stores, for each reception depth, the frequency values as upper limits of frequency bands in a plurality of reception depths;
a corrected frequency spectrum calculating step (S8) of calculating, by a corrected frequency spectrum calculator, a corrected frequency spectrum by correcting the frequency spectrum calculated at the frequency analyzing step (S5) based on a reference spectrum in the specified reception depth which is read out from a reference spectrum information storage unit that stores, for each reception depth, reference spectra in the plurality of reception depths obtained based on frequencies of ultrasonic waves received from a reference reflector; and
a feature data extracting step (S9, S10) of performing, by a feature data extracting unit, an approximating process only on a part of the corrected frequency spectrum calculated at the corrected frequency spectrum calculating step, the part of the corrected frequency spectrum being included in the frequency band set by the frequency band setting unit, to extract feature data indicating the tissue property of the subject using a result of the approximating process,

wherein the frequency band setting step includes setting of the frequency band which is used when the approximating process is performed.

**Patentansprüche**

1. Ultraschallbeobachtungsvorrichtung (1) zum Übertragen einer Ultraschallwelle an ein Subjekt, Empfangen der vom Subjekt reflektierten Ultraschallwelle und Bestimmen einer Gewebeeigenschaft des Subjekts auf der Grundlage eines Analyseergebnisses des Frequenzspektrums der empfangenen Ultraschallwelle, wobei die Ultraschallbeobachtungsvorrichtung (1) umfasst:

   einen Frequenzanalysator (41), der zur Berechnung eines Frequenzspektrums durch Analyse einer Frequenz der empfangenen Ultraschallwelle, die von einer spezifizierten Empfangstiefe empfangen wird, konfiguriert ist;
   eine Frequenzband-Informationsspeichereinheit (82), die für jede Empfangstiefe Frequenzwerte als obere Grenzwerte von Frequenzbändern in einer Vielzahl von Empfangstiefen speichert;
   eine Frequenzband-Einstellungseinheit (42), die konfiguriert ist, um ein Frequenzband des Frequenzspektrums in der spezifizierten Empfangstiefe einzustellen, das durch den Frequenzanalysator (41) berechnet wird, und zwar auf der Grundlage eines Frequenzwertes in der spezifizierten Empfangstiefe unter den in der Frequenzband-Informationsspeichereinheit (82) gespeicherten Frequenzwerten;
   eine Referenzspektrum-Informationsspeichereinheit (83), die für jede Empfangstiefe Referenzspektren in der Vielzahl von Empfangstiefen speichert, die auf der Grundlage von Frequenzen von von einem Referenzreflektor empfangenen Ultraschallwellen bezogen werden;
   einen Korrekturfrequenzspektrum-Rechner (43), der konfiguriert ist zur Berechnung eines korrigierten Frequenzspektrums durch Korrektur des Frequenzspektrums, das vom Frequenzanalysator (41) berechnet wird, und zwar auf der Grundlage des Referenzspektrums in der spezifizierten Empfangstiefe unter den in der Referenzspektrum-Informationsspeichereinheit (83) gespeicherten Referenzspektren; und
   eine Merkmalsdaten-Extraktionseinheit (44) enthaltend eine Approximationseinheit (441), die konfiguriert ist, um ein Approximationsverfahren nur an einem Teil des korrigierten Frequenzspektrums durchzuführen, das vom Korrekturfrequenzspektrum-Rechner (43) berechnet wird, wobei der Teil des korrigierten Frequenzspektrums im Frequenzband enthalten ist, das von der Frequenzband-Einstellungseinheit (42) eingestellt wird, und wobei die Merkmalsdaten-Extraktionseinheit (44) konfiguriert ist, um Merkmalsdaten zu extrahieren, die die Gewebeeigenschaft des Subjekts anzeigen, unter Verwendung eines Ergebnisses des von der Approximationseinheit (441) durchgeführten Approximationsverfahrens;
   wobei die Frequenzband-Einstellungseinheit (42) konfiguriert ist, um das Frequenzband einzustellen, das bei der Durchführung des Approximationsverfahrens durch die Approximationseinheit (441) verwendet wird.

2. Ultraschallbeobachtungsvorrichtung (1) nach Anspruch 1, wobei die Merkmalsdaten-Extraktionseinheit (44) einen Abschwächungskorrektor (442) umfasst, der konfiguriert ist zur Durchführung eines Abschwächungskorrekturverfahrens zur Durchführung einer identischen Transformation oder Reduktion eines Beitrags einer Abschwächung, die bei einer Übertragung der Ultraschallwelle je nach einer Empfangstiefe und einer Frequenz der Ultraschallwelle entsteht.

3. Ultraschallbeobachtungsvorrichtung (1) nach Anspruch 1, wobei die Frequenzband-Informationsspeichereinheit (82) weiterhin für jede Empfangstiefe Frequenzwerte als untere Grenzwerte der Frequenzbänder in der Vielzahl der Empfangstiefen speichert; und
   die Frequenzband-Einstellungseinheit (42) konfiguriert ist zur Einstellung des Frequenzbandes des Frequenzspektrums, das vom Frequenzanalysator berechnet wird, weiterhin basierend auf einem unteren Grenzwert eines Frequenzbandes in der spezifizierten Empfangstiefe, die in der Frequenzband-Informationsspeichereinheit (82) gespeichert ist.

4. Ultraschallbeobachtungsvorrichtung (1) nach Anspruch 3, weiterhin umfassend eine Ultraschallsonde (2) zur Übertragung und zum Empfang der Ultraschallwelle, wobei die Referenzspektrum-Informationsspeichereinheit (83) für jede Empfangstiefe entsprechend der Art der Ultraschallsonde (2) die Referenzspektren in der Vielzahl von Empfangstiefen speichert, die auf der Grundlage der Frequenzen der vom Referenzreflektor empfangenen Ultraschallwellen erhalten werden; und

die Frequenzband-Informationsspeichereinheit (82) für jede Empfangstiefe entsprechend der Art der Ultraschallsonde (2) die oberen Grenzwerte und unteren Grenzwerte der Frequenzbänder speichert.

5. Ultraschallbeobachtungsvorrichtung (1) nach Anspruch 2, wobei der Abschwächungskorrektor (442) konfiguriert, eine größere Korrektur durchzuführen, wenn eine Empfangstiefe der Ultraschallwelle größer ist.

6. Ultraschallbeobachtungsvorrichtung (1) nach einem der Ansprüche 1 und 5, wobei die Approximationseinheit (441) konfiguriert ist, um das Frequenzspektrum durch einen Polynomausdruck mittels einer Regressionsanalyse zu approximieren.

7. Ultraschallbeobachtungsvorrichtung (1) nach Anspruch 6, wobei die Approximationseinheit (441) konfiguriert ist zur Approximation des Frequenzspektrums durch einen primären Ausdruck, und mehrere Arten von Merkmalsdaten extrahiert, einschließlich mindestens zwei von einer Steigung des primären Ausdrucks, einem Achsenabschnitt des primären Ausdrucks und einer Intensität, die durch Verwendung der Steigung, des Achsenabschnitts und einer spezifischen, in einem Frequenzbandbereich des Frequenzspektrums enthaltenen Frequenz definiert ist.

8. Ultraschallbeobachtungsvorrichtung (1) nach Anspruch 1, weiterhin umfassend:

eine "Bekanntes-Subjekt"-Informationsspeichereinheit (81), die Merkmalsdaten von Frequenzspektren speichert, die auf der Grundlage von Ultraschallwellen extrahiert werden, die von einer Vielzahl von bekannten Subjekten reflektiert wurden, und zwar durch Zuordnung der Merkmalsdaten von Frequenzspektren zu Gewebeeigenschaften der Vielzahl von bekannten Subjekten; und
eine Gewebeeigenschafts-Bestimmungseinheit (45), die konfiguriert ist zur Bestimmung einer Gewebeeigenschaft in einem bestimmten Bereich des Subjekts (2) unter Verwendung der in der "Bekanntes-Subjekt"-Informationsspeichereinheit (81) mittels Zuordnung zur Vielzahl bekanntes Subjekt gespeicherten Merkmalsdaten sowie der Merkmalsdaten, die von der Merkmalsdaten-Extraktionseinheit (44) extrahiert wurden.

9. Ultraschallbeobachtungsvorrichtung (1) nach einem der Ansprüche 1 bis 8, wobei die Frequenzband-Informationsspeichereinheit (82) die Frequenzbänder speichert, für die gilt, dass je größer eine Empfangstiefe ist, desto schmaler eine Bandbreite ist und desto kleiner ein maximaler Frequenzwert ist, und die Bandbreite durch den maximalen Frequenzwert und einen minimalen Frequenzwert definiert ist.

10. Ultraschallbeobachtungsvorrichtung (1) nach einem der Ansprüche 1 bis 9, umfassend eine Anzeigeeinheit (7), die konfiguriert ist zur Erzeugung von visueller, den Merkmalsdaten des Subjekts entsprechender Information sowie zur Anzeige eines Bildes, das auf der Grundlage der erzeugten visuellen Information und der empfangenen Ultraschallwelle erzeugt wird.

11. Ultraschallbeobachtungsvorrichtung (1) nach Anspruch 10, wobei die visuelle Information eine Variable ist, die einen Farbraum darstellt.

12. Ultraschallbeobachtungsvorrichtung (1) nach Anspruch 2, wobei der Abschwächungskorrektor (442) zur Durchführung einer Korrektur auf der Grundlage eines Abschwächungsbetrags $A = 2\alpha z f$ konfiguriert ist, wobei "$\alpha$" eine Abschwächungsrate, "z" eine Empfangstiefe und "f" eine Frequenz angibt.

13. Betriebsverfahren für eine Ultraschallbeobachtungsvorrichtung zum Übertragen einer Ultraschallwelle an ein Subjekt, Empfangen der vom Subjekt reflektierten Ultraschallwelle und Bestimmen einer Gewebeeigenschaft des Subjekts auf der Grundlage eines Analyseergebnisses eines Frequenzspektrums der empfangenen Ultraschallwelle, wobei das Betriebsverfahren umfasst:

einen Frequenzanalyseschritt (S5) der durch einen Frequenzanalysator vorgenommenen Berechnung eines Frequenzspektrums durch Analyse einer Frequenz einer empfangenen Ultraschallwelle, die von einer spezifizierten Empfangstiefe empfangen wurde;
einen Frequenzband-Einstellungsschritt (S7) der durch eine Frequenzband-Einstellungseinheit vorgenommenen Einstellung eines Frequenzbandes des Frequenzspektrums in der spezifizierten Empfangstiefe, berechnet durch den Frequenzanalysator, und zwar auf der Grundlage eines Frequenzwertes in der spezifizierten Empfangstiefe unter den Frequenzwerten, die in einer Frequenzband-Informationsspeichereinheit gespeichert sind, die für jede Empfangstiefe die Frequenzwerte als obere Grenzwerte von Frequenzbändern in einer Vielzahl von Empfangstiefen speichert;

einen Korrekturfrequenzspektrum-Berechnungsschritt (S8) der von einem Korrekturfrequenzspektrum-Rechner vorgenommenen Berechnung eines korrigierten Frequenzspektrums durch Korrektur des Frequenzspektrums, das im Frequenzanalyseschritt berechnet wurde, und zwar auf der Grundlage eines Referenzspektrums in der spezifizierten Empfangstiefe, das aus einer Referenzspektrum-Informationsspeichereinheit ausgelesen wird, die für jede Empfangstiefe Referenzspektren in der Vielzahl von Empfangstiefen speichert, die basierend auf Frequenzen von von einem Referenzreflektor empfangenen Ultraschallwellen bezogen werden; und einen Merkmalsdaten-Extraktionsschritt (S9, S10) der durch eine Merkmalsdaten-Extraktionseinheit vorgenommenen Durchführung eines Approximationsverfahrens an nur einem Teil des korrigierten Frequenzspektrums, das im Korrekturfrequenzspektrum-Berechnungsschritt berechnet wird, wobei der Teil des korrigierten Frequenzspektrums im von der Frequenzband-Einstellungseinheit eingestellten Frequenzband enthalten ist, und zwar um Merkmalsdaten zu extrahieren, die die Gewebeeigenschaft des Subjekts unter Verwendung eines Ergebnisses des Approximationsverfahrens angeben, wobei der Frequenzband-Einstellungsschritt die Einstellung des Frequenzbandes umfasst, das bei Durchführung des Approximationsverfahrens verwendet wird.

14. Betriebsprogramm einer Ultraschallbeobachtungsvorrichtung zum Übertragen einer Ultraschallwelle an ein Subjekt, Empfangen der vom Subjekt reflektierten Ultraschallwelle und Bestimmen einer Gewebeeigenschaft des Subjekts auf der Grundlage eines Analyseergebnisses eines Frequenzspektrums der empfangenen Ultraschallwelle, wobei das Betriebsprogramm veranlasst, dass die Ultraschallbeobachtungsvorrichtung ausführt:

einen Frequenzanalyseschritt (S5) der durch einen Frequenzanalysator vorgenommenen Berechnung eines Frequenzspektrums durch Analyse einer Frequenz einer empfangenen Ultraschallwelle, die von einer spezifizierten Empfangstiefe empfangen wurde;
einen Frequenzband-Einstellungsschritt (S7) der durch eine Frequenzband-Einstellungseinheit vorgenommenen Berechnung eines Frequenzbandes des Frequenzspektrums in der spezifizierten Empfangstiefe, das im Frequenzanalyseschritt (S5) berechnet wird, und zwar auf der Grundlage eines Frequenzwertes in der spezifizierten Empfangstiefe unter den Frequenzwerten, die in einer Frequenzband-Informationsspeichereinheit gespeichert sind, die für jede Empfangstiefe die Frequenzwerte als obere Grenzwerte von Frequenzbändern in einer Vielzahl von Empfangstiefen speichert;
einen Korrekturfrequenzspektrum-Berechnungsschritt (S8) der durch einen Korrekturfrequenzspektrum-Rechner vorgenommenen Berechnung eines korrigierten Frequenzspektrums durch Korrektur des Frequenzspektrums, das im Frequenzanalyseschritt (S5) berechnet wird, und zwar auf der Grundlage eines Referenzspektrums in der spezifizierten Empfangstiefe, das aus einer Referenzspektrum-Informationsspeichereinheit ausgelesen wird, die für jede Empfangstiefe Referenzspektren in der Vielzahl von Empfangstiefen speichert, die basierend auf Frequenzen von von einem Referenzreflektor empfangenen Ultraschallwellen bezogen werden; und einen Merkmalsdaten-Extraktionsschritt (S9, S10) der durch eine Merkmalsdaten-Extraktionseinheit vorgenommenen Durchführung eines Approximationsverfahrens an nur einem Teil des korrigierten Frequenzspektrums, das im Korrekturfrequenzspektrum-Berechnungsschritt berechnet wird, wobei der Teil des korrigierten Frequenzspektrums im Frequenzband enthalten ist, das von der Frequenzband-Einstellungseinheit eingestellt wird, und zwar um Merkmalsdaten zu extrahieren, die die Gewebeeigenschaft des Subjekts unter Verwendung eines Ergebnisses des Approximationsverfahrens angeben, wobei der Frequenzband-Einstellungsschritt die Einstellung des Frequenzbandes umfasst, das bei Durchführung des Approximationsverfahrens verwendet wird.

## Revendications

1. Appareil d'observation échographique (1) pour émettre une onde ultrasonore vers un sujet, recevoir l'onde ultrasonore réfléchie par le sujet, et déterminer une propriété de tissu du sujet sur la base d'un résultat d'analyse du spectre de fréquence de l'onde ultrasonore reçue, l'appareil d'observation échographique (1) comprenant :

un analyseur de fréquence (41) configuré pour calculer un spectre de fréquence par analyse d'une fréquence de l'onde ultrasonore reçue qui est reçue en provenance d'une profondeur de réception spécifiée ;
une unité de stockage d'informations de bande de fréquences (82) qui stocke, pour chaque profondeur de réception, des valeurs de fréquence en tant que limites supérieures de bandes de fréquences dans une pluralité de profondeurs de réception ;
une unité de réglage de bande de fréquences (42) configurée pour régler une bande de fréquences du spectre de fréquence dans la profondeur de réception spécifiée, calculé par l'analyseur de fréquence (41), sur la base d'une valeur de fréquence dans la profondeur de réception spécifiée parmi les valeurs de fréquence stockées dans l'unité de stockage d'informations de bande de fréquences (82) ;

une unité de stockage d'informations de spectre de référence (83) qui stocke, pour chaque profondeur de réception, des spectres de référence dans la pluralité de profondeurs de réception obtenus sur la base de fréquences d'ondes ultrasonores reçues en provenance d'un réflecteur de référence ;

un calculateur de spectre de fréquence corrigé (43) configuré pour calculer un spectre de fréquence corrigé par correction du spectre de fréquence calculé par l'analyseur de fréquence (41) sur la base du spectre de référence dans la profondeur de réception spécifiée parmi les spectres de référence stockés dans l'unité de stockage d'informations de spectre de référence (83) ; et

une unité d'extraction de données de caractéristique (44) comprenant une unité d'approximation (441) configurée pour réaliser un traitement d'approximation uniquement sur une partie du spectre de fréquence corrigé calculé par le calculateur de spectre de fréquence corrigé (43), la partie du spectre de fréquence corrigé étant comprise dans la bande de fréquences réglée par l'unité de réglage de bande de fréquences (42), l'unité d'extraction de données de caractéristique (44) étant configurée pour extraire des données de caractéristique indiquant la propriété de tissu du sujet à l'aide d'un résultat du traitement d'approximation réalisé par l'unité d'approximation (441) ;

l'unité de réglage de bande de fréquences (42) étant configurée pour régler la bande de fréquences qui est utilisée lorsque l'unité d'approximation (441) réalise le traitement d'approximation.

2. Appareil d'observation échographique (1) selon la revendication 1, dans lequel l'unité d'extraction de données de caractéristique (44) comprend un correcteur d'atténuation (442) configuré pour réaliser un traitement de correction d'atténuation afin de réaliser une transformation identique ou de réduire une contribution d'une atténuation qui survient dans une transmission d'onde ultrasonore en fonction d'une profondeur de réception et d'une fréquence de l'onde ultrasonore.

3. Appareil d'observation échographique (1) selon la revendication 1, dans lequel l'unité de stockage d'informations de bande de fréquences (82) stocke en outre, pour chaque profondeur de réception, des valeurs de fréquence en tant que limites inférieures des bandes de fréquences dans la pluralité de profondeurs de réception, et

l'unité de réglage de bande de fréquences (42) est configurée pour régler la bande de fréquences du spectre de fréquence calculé par l'analyseur de fréquence, sur la base en outre d'une limite inférieure d'une bande de fréquences dans la profondeur de réception spécifiée stockée dans l'unité de stockage d'informations de bande de fréquences (82).

4. Appareil d'observation échographique (1) selon la revendication 3, comprenant en outre une sonde à ultrasons (2) pour émettre et recevoir l'onde ultrasonore, l'unité de stockage d'informations de spectre de référence (83) stockant, pour chaque profondeur de réception selon le type de sonde à ultrasons (2), les spectres de référence dans la pluralité de profondeurs de réception obtenus sur la base des fréquences des ondes ultrasonores reçues en provenance du réflecteur de référence, et

l'unité de stockage d'informations de bande de fréquences (82) stockant, pour chaque profondeur de réception selon le type de sonde à ultrasons (2), les limites supérieures et les limites inférieures des bandes de fréquences.

5. Appareil d'observation échographique (1) selon la revendication 2, dans lequel le correcteur d'atténuation (442) est configuré pour réaliser une plus grande correction à mesure qu'une profondeur de réception de l'onde ultrasonore augmente.

6. Appareil d'observation échographique (1) selon l'une quelconque des revendications 1 et 5, dans lequel l'unité d'approximation (441) est configurée pour approximer le spectre de fréquence par une expression polynomiale par l'intermédiaire d'une analyse de régression.

7. Appareil d'observation échographique (1) selon la revendication 6, dans lequel l'unité d'approximation (441) est configurée pour approximer le spectre de fréquence par une expression primaire et extraire plusieurs types de données de caractéristique comprenant au moins deux parmi une pente de l'expression primaire, un point d'intersection de l'expression primaire, et une intensité qui est définie en utilisant la pente, le point d'intersection et une fréquence spécifique comprise dans une plage de bandes de fréquences du spectre de fréquence.

8. Appareil d'observation échographique (1) selon la revendication 1, comprenant en outre :

une unité de stockage d'informations de sujet connu (81) qui stocke des données de caractéristique de spectres de fréquence extraites sur la base d'ondes ultrasonores réfléchies par une pluralité de sujets connus, en associant les données de caractéristique de spectres de fréquence à des propriétés de tissu de la pluralité de sujets connus ; et

une unité de détermination de propriété de tissu (45) configurée pour déterminer une propriété de tissu dans une zone spécifiée du sujet (2) en utilisant les données de caractéristique, stockées dans l'unité de stockage d'informations de sujet connu (81) en étant associées à la pluralité de sujets connus, et les données de caractéristique extraites par l'unité d'extraction de données de caractéristique (44).

9. Appareil d'observation échographique (1) selon l'une quelconque des revendications 1 à 8, dans lequel l'unité de stockage d'informations de bande de fréquences (82) stocke les bandes de fréquences dans lesquelles plus la profondeur de réception est grande, plus la largeur de bande est étroite et plus la valeur de fréquence maximale est petite, et la largeur de bande étant définie par la valeur de fréquence maximale et une valeur de fréquence minimale.

10. Appareil d'observation échographique (1) selon l'une quelconque des revendications 1 à 9, comprenant une unité d'affichage (7) configurée pour générer des informations visuelles correspondant aux données de caractéristique du sujet et afficher une image générée sur la base des informations visuelles générées et de l'onde ultrasonore reçue.

11. Appareil d'observation échographique (1) selon la revendication 10, dans lequel les informations visuelles sont une variable constituant un espace de couleur.

12. Appareil d'observation échographique (1) selon la revendication 2, dans lequel le correcteur d'atténuation (442) est configuré pour réaliser une correction sur la base d'une quantité d'atténuation $A = 2\alpha zf$, où « $\alpha$ » indique un taux d'atténuation, « z » indique une profondeur de réception, et « f » indique une fréquence.

13. Procédé de fonctionnement d'un appareil d'observation échographique pour émettre une onde ultrasonore vers un sujet, recevoir l'onde ultrasonore réfléchie par le sujet, et déterminer une propriété de tissu du sujet sur la base d'un résultat d'analyse d'un spectre de fréquence de l'onde ultrasonore reçue, le procédé de fonctionnement comprenant :

une étape d'analyse de fréquence (S5) consistant à calculer, par un analyseur de fréquence, un spectre de fréquence par analyse d'une fréquence de l'onde ultrasonore reçue qui est reçue en provenance d'une profondeur de réception spécifiée ;

une étape de réglage de bande de fréquences (S7) consistant à régler, par une unité de réglage de bande de fréquences, une bande de fréquences du spectre de fréquence dans la profondeur de réception spécifiée, calculé par l'analyseur de fréquence, sur la base d'une valeur de fréquence dans la profondeur de réception spécifiée parmi des valeurs de fréquence stockées dans une unité de stockage d'informations de bande de fréquences qui stocke, pour chaque profondeur de réception, les valeurs de fréquence en tant que limites supérieures de bandes de fréquences dans une pluralité de profondeurs de réception ;

une étape de calcul de spectre de fréquence corrigé (S8) consistant à calculer, par un calculateur de spectre de fréquence corrigé, un spectre de fréquence corrigé par correction du spectre de fréquence calculé dans l'étape d'analyse de fréquence sur la base d'un spectre de référence dans la profondeur de réception spécifiée qui est lu à partir d'une unité de stockage d'informations de spectre de référence qui stocke, pour chaque profondeur de réception, des spectres de référence dans la pluralité de profondeurs de réception obtenus sur la base de fréquences d'ondes ultrasonores reçues en provenance d'un réflecteur de référence ; et

une étape d'extraction de données de caractéristique (S9, S10) consistant à réaliser, par une unité d'extraction de données de caractéristique, un traitement d'approximation uniquement sur une partie du spectre de fréquence corrigé calculé dans l'étape de calcul de spectre de fréquence corrigé, la partie du spectre de fréquence corrigé étant comprise dans la bande de fréquences réglée par l'unité de réglage de bande de fréquences, pour extraire des données de caractéristique indiquant la propriété de tissu du sujet à l'aide d'un résultat du traitement d'approximation,

l'étape de réglage de bande de fréquences comprenant régler la bande de fréquences qui est utilisée lorsque le traitement d'approximation est réalisé.

14. Programme de fonctionnement d'un appareil d'observation échographique pour émettre une onde ultrasonore vers un sujet, recevoir l'onde ultrasonore réfléchie par le sujet, et déterminer une propriété de tissu du sujet sur la base d'un résultat d'analyse d'un spectre de fréquence de l'onde ultrasonore reçue, le programme de fonctionnement amenant l'appareil d'observation échographique à exécuter :

**EP 2 599 440 B1**

une étape d'analyse de fréquence (S5) consistant à calculer, par un analyseur de fréquence, un spectre de fréquence par analyse d'une fréquence d'une onde ultrasonore reçue qui est reçue en provenance d'une profondeur de réception spécifiée ;

une étape de réglage de bande de fréquences (S7) consistant à régler, par une unité de réglage de bande de fréquences, une bande de fréquences du spectre de fréquence dans la profondeur de réception spécifiée, calculé dans l'étape d'analyse de fréquence (S5), sur la base d'une valeur de fréquence dans la profondeur de réception spécifiée parmi des valeurs de fréquence stockées dans une unité de stockage d'informations de bande de fréquences qui stocke, pour chaque profondeur de réception, les valeurs de fréquence en tant que limites supérieures de bandes de fréquences dans une pluralité de profondeurs de réception ;

une étape de calcul de spectre de fréquence corrigé (S8) consistant à calculer, par un calculateur de spectre de fréquence corrigé, un spectre de fréquence corrigé par correction du spectre de fréquence calculé dans l'étape d'analyse de fréquence (S5) sur la base d'un spectre de référence dans la profondeur de réception spécifiée qui est lu à partir d'une unité de stockage d'informations de spectre de référence qui stocke, pour chaque profondeur de réception, des spectres de référence dans la pluralité de profondeurs de réception obtenus sur la base de fréquences d'ondes ultrasonores reçues en provenance d'un réflecteur de référence ; et

une étape d'extraction de données de caractéristique (S9, S10) consistant à réaliser, par une unité d'extraction de données de caractéristique, un traitement d'approximation uniquement sur une partie du spectre de fréquence corrigé calculé dans l'étape de calcul de spectre de fréquence corrigé, la partie du spectre de fréquence corrigé étant comprise dans la bande de fréquences réglée par l'unité de réglage de bande de fréquences, pour extraire des données de caractéristique indiquant la propriété de tissu du sujet à l'aide d'un résultat du traitement d'approximation,

l'étape de réglage de bande de fréquences comprenant régler la bande de fréquences qui est utilisée lorsque le traitement d'approximation est réalisé.

# FIG.1

ULTRASONIC PROBE $\sim 2$
- SIGNAL CONVERTER $\sim 21$

TRANSCEIVER $\sim 3$

INPUT UNIT $\sim 6$

$1$

CONTROL UNIT $\sim 9$

STORAGE UNIT $\sim 8$
- KNOWN SUBJECT INFORMATION STORAGE UNIT $\sim 81$
- FREQUENCY BAND INFORMATION STORAGE UNIT $\sim 82$
- REFERENCE SPECTRUM INFORMATION STORAGE UNIT $\sim 83$
- WINDOW FUNCTION STORAGE UNIT $\sim 84$
- CORRECTION INFORMATION STORAGE UNIT $\sim 85$

DISPLAY UNIT $\sim 7$

OPERATION UNIT $\sim 4$
- FREQUENCY ANALYZER $\sim 41$
- FREQUENCY BAND SETTING UNIT $\sim 42$
- CORRECTED FREQUENCY SPECTRUM CALCULATOR $\sim 43$
- FEATURE DATA EXTRACTING UNIT $\sim 44$
  - APPROXIMATING UNIT $\sim 441$
  - ATTENUATION CORRECTOR $\sim 442$
- TISSUE PROPERTY DETERMINING UNIT $\sim 45$

IMAGE PROCESSOR $\sim 5$
- B-MODE IMAGE DATA GENERATOR $\sim 51$
- DETERMINATION RESULT DISPLAYING IMAGE DATA GENERATOR $\sim 52$

# FIG.2

| RECEPTION DEPTH (cm) | $f_{LOW}$ (MHz) | $f_{HIGH}$ (MHz) |
|---|---|---|
| 2 | 4 | 9 |
| 4 | 4 | 9 |
| 6 | 4 | 9 |
| 8 | 3.5 | 8 |
| 10 | 3 | 6.5 |
| 12 | 2.5 | 5 |

Tb

# FIG.3

# FIG.4

```
                    ┌─────────────┐
                    │    START    │
                    └──────┬──────┘
                           ▼
        ┌──────────────────────────────┐
        │    MEASURE NEW SUBJECT        │── S1
        └──────────────┬───────────────┘
                       ▼
        ┌──────────────────────────────┐
        │  GENERATE B-MODE IMAGE DATA   │── S2
        └──────────────┬───────────────┘
                       ▼
        ┌──────────────────────────────┐
        │    DISPLAY B-MODE IMAGE       │── S3
        └──────────────┬───────────────┘
                       ▼
              ◇ S4                      NO
        ╱ IS AREA OF ╲ ─────────────────────┐
        ╲ INTEREST SET? ╱                    │
              ▼ YES                          ▼  S6      NO
        ┌──────────────────┐            ◇ END? ◇ ──────┐
        │ PERFORM FREQUENCY │── S5        ▼ YES          │
        │    ANALYSIS       │                           │
        └─────────┬─────────┘                           │
                  ▼                                     │
        ┌──────────────────┐                           │
        │ SET FREQUENCY BAND │── S7                      │
        └─────────┬─────────┘                           │
                  ▼                                     │
        ┌──────────────────┐                           │
        │ CALCULATE CORRECTED │── S8                     │
        │ FREQUENCY SPECTRUM  │                          │
        └─────────┬─────────┘                           │
                  ▼                                     │
        ┌──────────────────┐                           │
        │ EXTRACT BEFORE-CORRECTION │── S9               │
        │    FEATURE DATA    │                          │
        └─────────┬─────────┘                           │
                  ▼                                     │
        ┌──────────────────┐                           │
        │ PERFORM ATTENUATION │── S10                    │
        │ CORRECTION ON BEFORE- │                        │
        │ CORRECTION FEATURE DATA │                      │
        └─────────┬─────────┘                           │
                  ▼                                     │
        ┌──────────────────┐                           │
        │ DETERMINE TISSUE PROPERTY │── S11              │
        └─────────┬─────────┘                           │
                  ▼                                     │
        ┌──────────────────┐                           │
        │ GENERATE DETERMINATION │── S12                 │
        │ RESULT DISPLAYING IMAGE DATA │                 │
        └─────────┬─────────┘                           │
                  ▼                                     │
        ┌──────────────────┐                           │
        │ DISPLAY DETERMINATION RESULT │── S13           │
        │   DISPLAYING IMAGE  │                          │
        └─────────┬─────────┘                           │
                  ▼◄────────────────────────────────────┘
            ┌─────────────┐
            │     END     │
            └─────────────┘
```

27

# FIG.5

100

# FIG.6

```
        ⟨ FREQUENCY ⟩
        ⟨ ANALYSIS  ⟩
             │
             ▼
        ┌─────────────────┐
        │    L = L₀       │──── S21
        └─────────────────┘
             │
    ┌────────┤
    │        ▼
    │   ┌─────────────────┐
    │   │    Z = Z₀       │──── S22
    │   └─────────────────┘
    │        │
    │   ┌────┤
    │   │    ▼
    │   │ ┌──────────────────────┐
    │   │ │ OBTAIN FFT DATA GROUP │──── S23
    │   │ └──────────────────────┘
    │   │    │
    │   │    ▼
    │   │ ┌──────────────────────┐
    │   │ │ MAKE WINDOW FUNCTION  │──── S24
    │   │ │        WORK           │
    │   │ └──────────────────────┘
    │   │    │
    │   │    ▼
    │   │        S25
    │   │   ◇──────────◇     NO
    │   │   ╱ IS FFT DATA ╲ ─────────┐
    │   │   ╲ GROUP NORMAL?╱         │
    │   │   ◇──────────◇          ┌──────────────────────────┐  S26
    │   │     │ YES               │ INSERT ZERO FOR DEFICIENCY│
    │   │     │◄──────────────────└──────────────────────────┘
    │   │     ▼
    │   │ ┌──────────────────────┐
    │   │ │ PERFORM FFT OPERATION │──── S27
    │   │ └──────────────────────┘
    │   │     │
    │   │     ▼
    │   │ ┌──────────────────────┐
    │   │ │      Z = Z+D          │──── S28
    │   │ └──────────────────────┘
    │   │     │
    │   │     ▼        S29
    │   │   ◇──────────◇   NO
    │   └───╲ Z > Zmax? ╱─────
    │       ◇──────────◇
    │         │ YES
    │         ▼
    │   ┌──────────────────────┐
    │   │      L = L+1          │──── S30
    │   └──────────────────────┘
    │         │
    │         ▼        S31
    │       ◇──────────◇
    │   NO  ╲ L > Lmax? ╱
    └───────◇──────────◇
              │ YES
              ▼
          ⟨ RETURN ⟩
```

**L = L₀** — S21 → $L = L_0$

**Z = Z₀** — S22 → $Z = Z_0$

**OBTAIN FFT DATA GROUP** — S23

**MAKE WINDOW FUNCTION WORK** — S24

**IS FFT DATA GROUP NORMAL?** — S25

**INSERT ZERO FOR DEFICIENCY** — S26

**PERFORM FFT OPERATION** — S27

**Z = Z+D** — S28 → $Z = Z+D$

**Z > Z_max?** — S29 → $Z > Z_{max}?$

**L = L+1** — S30 → $L = L+1$

**L > L_max?** — S31 → $L > L_{max}?$

# FIG.7

# FIG.8

# FIG.9

# FIG.10

# FIG.11

# FIG.12

# FIG.13

# FIG.14

# FIG.15

# FIG.16

# FIG.17

```
┌─────────────────────────────┐
│   TISSUE PROPERTY           │
│   DETERMINATION             │
└─────────────────────────────┘
              │
              ▼
┌─────────────────────────────┐
│   SET FEATURE DATA SPACE    │────S41
└─────────────────────────────┘
              │
              ▼
┌─────────────────────────────┐
│ CALCULATE DISTANCE ON FEATURE│
│ DATA SPACE BETWEEN SUBJECT POINT│──S42
│ AND KNOWN SUBJECT AVERAGE POINT│
│      OF EACH GROUP          │
└─────────────────────────────┘
              │
              ▼
┌─────────────────────────────┐
│  DETERMINE TISSUE PROPERTY  │────S43
└─────────────────────────────┘
              │
              ▼
┌─────────────────────────────┐
│  OUTPUT DETERMINATION RESULT │────S44
└─────────────────────────────┘
              │
              ▼
┌─────────────────────────────┐
│         RETURN              │
└─────────────────────────────┘
```

# FIG.18

# FIG.19

201

202    200

IDENTIFYING
INFORMATION
ID
NAME
SEX

TISSUE PROPERTY
DETERMINATION RESULT

INFORMATION OF
FEATURE DATA

INFORMATION OF
ULTRASONIC IMAGE
QUALITY
:

300

# FIG.20

400

300

FIG.21

```
                    ┌─────────────┐
                    │    START    │
                    └──────┬──────┘
                           │
                           ▼
         ┌──────────────────────────────┐
         │     MEASURE NEW SUBJECT       │────S51
         └───────────────┬──────────────┘
                         │
                         ▼
         ┌──────────────────────────────┐
         │   GENERATE B-MODE IMAGE DATA  │────S52
         └───────────────┬──────────────┘
                         │
                         ▼
         ┌──────────────────────────────┐
         │      DISPLAY B-MODE IMAGE     │────S53
         └───────────────┬──────────────┘
                         │
                         ▼
                     ╱───────╲  S54
                   ╱  IS AREA   ╲        NO
                 ╱  OF INTEREST  ╲───────────────┐
                 ╲     SET?      ╱                │
                   ╲           ╱                  │
                     ╲───────╱                    ▼
                         │ YES              ╱─────────╲  S56
                         ▼                ╱    END?    ╲  NO
         ┌──────────────────────────────┐ ╲           ╱───┐
         │     PERFORM FREQUENCY         │────S55  ╲─────╱    │
         │         ANALYSIS              │           │ YES    │
         └───────────────┬──────────────┘           │        │
                         │                           │        │
                         ▼                           │        │
         ┌──────────────────────────────┐           │        │
         │      SET FREQUENCY BAND       │────S57    │        │
         └───────────────┬──────────────┘           │        │
                         │                           │        │
                         ▼                           │        │
         ┌──────────────────────────────┐           │        │
         │    CALCULATE CORRECTED        │────S58    │        │
         │    FREQUENCY SPECTRUM         │           │        │
         └───────────────┬──────────────┘           │        │
                         │                           │        │
                         ▼                           │        │
         ┌──────────────────────────────┐           │        │
         │   PERFORM ATTENUATION         │           │        │
         │ CORRECTION ON CORRECTED       │────S59    │        │
         │    FREQUENCY SPECTRUM         │           │        │
         └───────────────┬──────────────┘           │        │
                         │                           │        │
                         ▼                           │        │
         ┌──────────────────────────────┐           │        │
         │     EXTRACT FEATURE DATA      │────S60    │        │
         └───────────────┬──────────────┘           │        │
                         │                           │        │
                         ▼                           │        │
         ┌──────────────────────────────┐           │        │
         │   DETERMINE TISSUE PROPERTY   │────S61    │        │
         └───────────────┬──────────────┘           │        │
                         │                           │        │
                         ▼                           │        │
         ┌──────────────────────────────┐           │        │
         │  GENERATE DETERMINATION       │────S62    │        │
         │ RESULT DISPLAYING IMAGE DATA  │           │        │
         └───────────────┬──────────────┘           │        │
                         │                           │        │
                         ▼                           │        │
         ┌──────────────────────────────┐           │        │
         │ DISPLAY DETERMINATION RESULT  │────S63    │        │
         │     DISPLAYING IMAGE          │           │        │
         └───────────────┬──────────────┘           │        │
                         │                           │        │
                         ▼◄──────────────────────────┘        │
                    ┌─────────────┐                           │
                    │     END     │                           │
                    └─────────────┘                           │
```

# FIG.22

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005122906 A
- US 20070006651 A
- CA 2511629
- US 20100249590 A
- US 20060064014 A